(19) Europäisches Patentamt
European Patent Office
Office européen des brevets

(11) EP 0 787 485 B1

(12) **FASCICULE DE BREVET EUROPEEN**

(45) Date de publication et mention de la délivrance du brevet:
**25.11.1998 Bulletin 1998/48**

(51) Int. Cl.$^6$: **A61K 7/48**, A61K 7/06

(21) Numéro de dépôt: **97400218.0**

(22) Date de dépôt: **30.01.1997**

(54) **Emulsion huile-dans-eau comprenant un tensioactif siliconé anionique, composition comprenant une telle émulsion et utilisation en cosmétique, en pharmacie ou en hygiène**

Öl-in-Wasser-Emulsion, die eine anionische Silikontenside enthält, die Emulsion enthaltende Zusammensetzung und ihre Verwendung in Kosmetik, Pharmazie und Hygiene

Oil-in-water emulsion containing an anionic silicone surfactant, composition containing the same and its use in cosmetics, pharmacy or hygiene

(84) Etats contractants désignés:
**DE ES FR GB IT**

(30) Priorité: **07.02.1996 FR 9601514**

(43) Date de publication de la demande:
**06.08.1997 Bulletin 1997/32**

(73) Titulaire: **L'OREAL**
**75008 Paris (FR)**

(72) Inventeurs:
- **Terren, Nadia**
  **94550 Chevilly Larue (FR)**
- **Favre, Sophie**
  **94550 Chevilly Larue (FR)**

(74) Mandataire: **Dodin, Catherine**
**L'Oreal-D.P.I.,**
**90, rue du Général Roguet**
**92583 Clichy Cédex (FR)**

(56) Documents cités:
**EP-A- 0 576 189**
**WO-A-93/25179**
**WO-A-94/14822**
**US-A- 5 280 099**
**US-A- 5 286 830**
**US-A- 5 382 381**
- **Larousse 1994**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 787 485 B1

Printed by Xerox (UK) Business Services
2.16.6/3.4

## Description

L'invention concerne une émulsion huile-dans-eau, une composition et leur application en cosmétique, en pharmacie ou en hygiène.

Les compositions de fond de teint se présentent généralment sous forme de crème plus ou moins fluide comprenant des corps gras tels que des huiles et une phase particulaire généralement composée de charges et de pigments. Ces compositions, lorsqu'elles sont appliquées sur la peau, présentent toutefois l'inconvénient de transférer, c'est-à-dire de se déposer au moins en partie, en laissant une trace, sur certains supports avec lesquels elles peuvent être mises en contact, et notamment un vêtement ou la peau. Il s'en suit une persistance médiocre du film sur la peau d'où la nécessité de renouveler régulièrement l'application de la composition de fond de teint.
Un autre inconvénient des compositions de l'art antérieur réside dans le problème de migration de ces compositions, c'est-à-dire dans le fait que la composition a tendance à se propager à l'intérieur des plis et/ou des rides du visage, en créant un effet inesthétique.

La présente invention a pour but de pallier ces inconvénients et propose une émulsion qui présente une très bonne tenue ainsi qu'une bonne persistance sur la peau.

Il est connu des documents US 5,382,381 et WO93/25179 des émulsions huile-dans-eau comprenant un tensioactif siliconé comprenant au moins un groupement anionique.

Il est également connu du document WO94/14822 des compositions comprenant des agents gélifiants des huiles siliconées.

Il est de plus connu de EP-A-576 189 des compositions autobronzantes comprenant un copolyol siliconé anionique.

Le demandeur a découvert de façon surprenante et inattendue qu'en utilisant un tensioactif siliconé particulier, en association avec un choix d'huiles particulières, on peut obtenir une émulsion huile-dans-eau présentant lesdites caractéristiques, et qui présente également l'avantage de ne pas transférer.

Plus précisément, l'invention se rapporte à l'utilisation de l'association d'un tensioactif siliconé comprenant au moins un groupement anionique et d'une phase grasse comprenant au moins une première huile choisie parmi les huiles volatiles, les polyalkyl($C_1$-$C_{20}$)siloxanes et leurs mélanges dans une émulsion huile-dans-eau pour diminuer le transfert et/ou la migration et/ou pour améliorer la persistance et/ou la tenue de ladite émulsion ou d'une composition la comprenant, sur la peau.

Un autre objet de l'invention concerne l'utilisation de l'association d'un tensioactif siliconé comprenant au moins un groupement anionique et d'une phase grasse comprenant une première huile choisie parmi les huiles volatiles, les polyalkyl($C_1$-$C_{20}$)siloxanes et leurs mélanges, dans une émulsion huile-dans-eau qui ne transfère pas et/ou qui ne migre pas sur la peau.
Un autre objet de l'invention consiste en une émulsion huile-dans-eau caractérisée par le fait qu'elle comprend :

- (a) une phase aqueuse,
- (b) une phase grasse comprenant au moins une première huile choisie parmi les huiles volatiles, les polyalkyl($C_1$-$C_{20}$)siloxanes, et leurs mélanges, à une teneur d'au moins 65 % en poids par rapport au poids total de la phase grasse,
- (c) un tensioactif siliconé comprenant au moins un groupement anionique choisi parmi les tensioactifs définis ci-après,
  sous réserve que lorsque le tensioactif siliconé est de formule (I), la phase grasse de l'émulsion comprend un autre corps gras, autre que ladite première huile, à une teneur inférieure ou égale à 7 % en poids par rapport au poids total de l'émulsion.

Un autre objet de l'invention concerne une composition, en particulier cosmétique, pharmaceutique ou hygiénique, comprenant une émulsion telle que définie ci-dessus.

L'invention porte également sur un procédé de traitement non thérapeutique de la Peau et/ou du cuir chevelu, caractérisé par le fait qu'on applique sur la peau et/ou sur le cuir chevelu une émulsion et/ou une composition telles que définies ci-dessus.

L'invention concerne également un procédé de maquillage de la peau et/ou du cuir chevelu, caractérisé par le fait qu'on applique sur la peau et/ou sur le cuir chevelu une émulsion et/ou une composition telles que définies ci-dessus.

De préférence, dans lesdites utilisations, la teneur en ladite première huile dans la phase grasse de l'émulsion est d'au moins 65 % en poids par rapport au poids total de ladite phase grasse.

On a également constaté que l'émulsion utilisée selon l'invention s'applique et s'étale facilement de façon homogène, sans laisser de sensation de gras et présente de bonnes propriétés cosmétiques. Le film obtenu présente également une texture légère et reste confortable à porter tout au long de la journée.
De plus, l'émulsion appliquée sur la peau présente l'avantage de ne pas migrer dans les plis de la peau et/ou les rides

du visage.
Par ailleurs, il est possible d'ajouter à l'émulsion selon l'invention d'autres additifs tels que des huiles et/ou des poudres (pigments et/ou charges) tout en conservant une émulsion stable. L'émulsion est donc compatible avec un grand nombre d'adjuvants cosmétiques.
Enfin, on a constaté que la viscosité de l'émulsion est stable dans le temps.

L'émulsion utilisée selon l'invention comprend donc un tensioactif siliconé qui comprend au moins un groupement anionique.
Ledit tensioactif peut donc être un tensioactif amphotère ou, de préférence, un tensioactif anionique.

Selon l'invention, le groupement anionique présent dans le tensioactif siliconé peut être choisi parmi les groupements phosphate, sulfate, sulfonate et/ou carboxylate.

Parmi les tensioactifs siliconés à groupement phosphate, on peut citer ceux de formules (I) à (IV) suivantes :

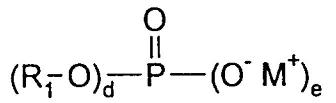
(I)

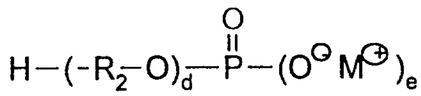
(II)

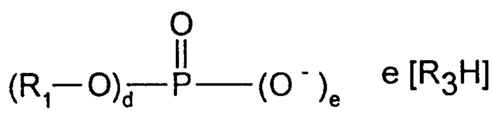
(III)

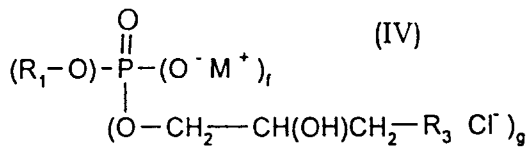
(IV)

formules dans lesquelles :

- $R_1$ désigne le radical de formule (V) suivante :

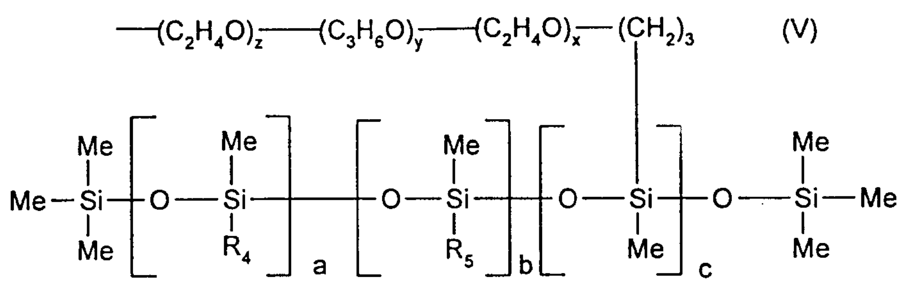
(V)

- $R_2$ est le radical de formule (VI) suivante :

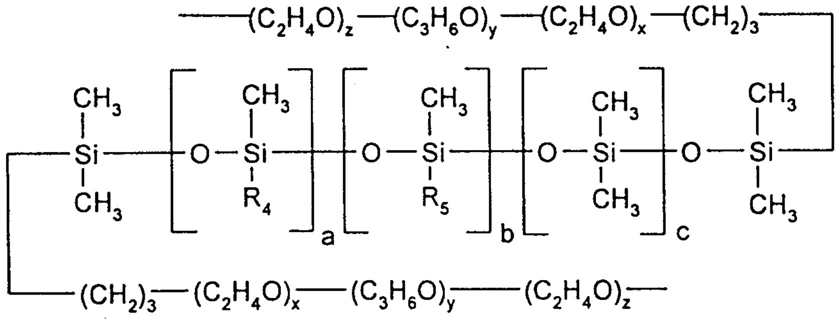

(VI)

dans lesquelles :

- . Me désigne le radical méthyle,
- . $C_2H_4O$ représente le groupement $-CH_2-CH_2-O-$,
- . $C_3H_6O$ représente le groupement $-CH_2-CH(CH_3)-O-$
- . a est un nombre entier allant de 0 à 200,
- . b est un nombre entier allant de 0 à 200,
- . c est un nombre entier allant de 1 à 200,
- . $R_4$ désigne un radical $-(CH_2)_nCH_3$ ou phényle, n étant un nombre entier allant de 0 à 10, et de préférence $R_4$ désigne un radical méthyle
- . $R_5$ est un radical $-(CH_2)_3-(OCH_2CH_2)_x-(OCH_2CH(CH_3))_y-(OCH_2CH_2)_z-OH$, . x, y, z sont des nombres entiers, allant indépendamment de 0 à 20, et de préférence $x+y+z \geq 3$ ,

- d et e vont de 1 à 2, avec d+e=3 ,
- f est égal à 0 ou 1, g est égal à 1 ou 2, avec f+g=2 ,
- M est choisi dans le groupe constitué de H, Na, K, Li, $NH_4$ et $N(CH_2CH_2OH)_3$
- $R_3$ est choisi parmi :

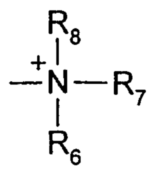

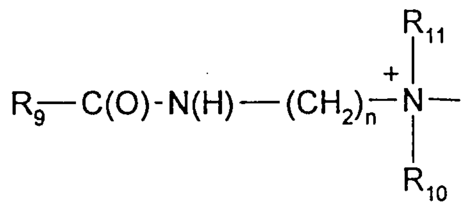

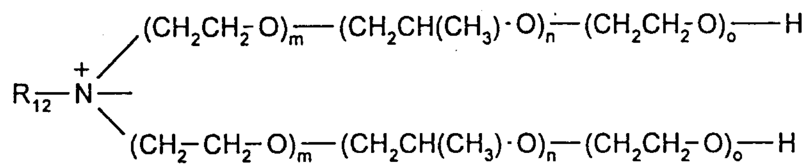

et

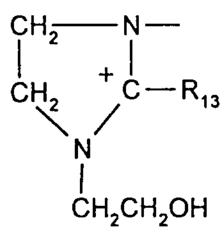

formules dans lesquelles :

- $R_6$ à $R_9$ désignent, indépendamment, un radical alkyle ayant de 1 à 20 atomes de carbone,
- $R_{10}$ et $R_{11}$ désignent, indépendamment, un radical alkyle ayant de 1 à 3 atomes de carbone,
- $R_{12}$ et $R_{13}$ désignent, indépendamment, un radical alkyle ayant de 6 à 20 atomes de carbone,
- m, n et o désignent, indépendamment un nombre entier allant de 0 à 20.

Les tensioactifs de formule (I) sont notamment décrits dans le brevet US-A-5 070 171 et sont commercialisés sous les dénominations "PECOSIL PS-100", "PECOSIL PS-200" et "PECOSIL WDS-100" par la société PHOENIX CHEMICAL.

Les tensioactifs de formule (II), (III) et (IV) sont notamment décrits respectivement dans les brevets US-A-5149765, US-A-5093452 et US-A-5091493.

Parmi les tensioactifs siliconés à groupement phosphate, on utilise de préférence ceux de formule (I).

Parmi les tensioactifs siliconés à groupement sulfate, on peut citer ceux de formule (VII) suivante :

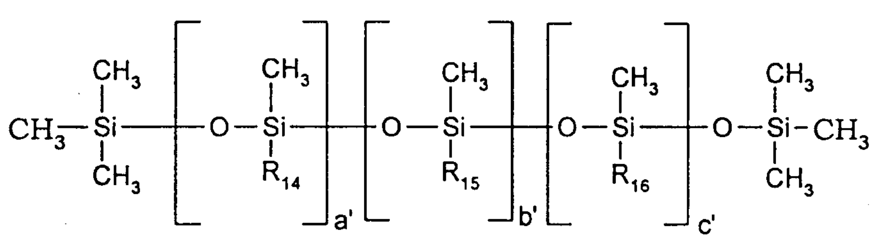
(VII)

dans laquelle

- $R_{14}$ désigne un radical alkyle ayant de 1 à 8 atomes de carbone ou un radical phényle,
- $R_{15}$ désigne un radical-$(CH_2)_3$-O-$(CH_2CH_2O)_u$-$(CH_2CH(CH_3)$-$O)_v$-$(CH_2CH_2O)_w$-$SO_3^-M^+$, M étant choisi parmi Na,

K, Li et $NH_4$,

- . $R_{16}$ désigne un radical $-(CH_2)_3-O-(CH_2CH_2O)_u-(CH_2CH(CH_3)-O)_v-(CH_2CH_2O)_w-H$, dans lesquels u, v, w désignent, indépendamment, un nombre entier allant de 0 à 100,
- . a' et c' désignent, indépendamment, un nombre entier allant de 0 à 50,
- . b' désigne un nombre entier allant de 1 à 50, et de préférence c' = 0.

Les composés de formule (VII) sont notamment décrits dans le brevet US-A-4 960 845

Parmi les tensioactifs siliconés à groupement sulfonate, on peut citer ceux obtenus par la réaction d'une silicone de formule (VIII) :

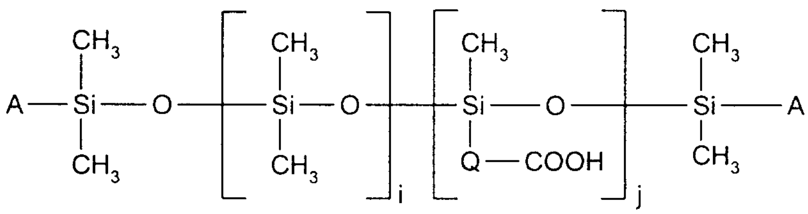
(VIII)

dans laquelle

- . Q désigne $(CH_2)_r$, r étant un nombre entier allant de 3 à 17,
- . j désigne soit un nombre entier allant de 1 à 10 et A désigne un radical méthyle, soit j= 0 et A désigne un radical -Q-COOH,
- . i désigne un nombre entier allant de 1 à 200,

avec un dérivé de taurine de formule $R_{17}-NH-(CH_2)_2-SO_3M$, dans laquelle

- . $R_{17}$ désigne un radical alkyle ayant de 1 à 40 atomes de carbone, et
- . M est choisi parmi Na, K, Li et $NH_4$.

Les composés ainsi préparés sont décrits dans le brevet US-A- 5 286 830.

Comme tensioactifs siliconés à groupement sulfonate, on peut également citer ceux de formule (IX) suivante :

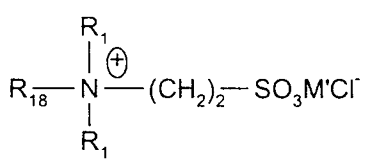
(IX)

dans laquelle

- . $R_{18}$ désigne un radical alkyle ayant de 1 à 40 atomes de carbone,
- . $R_1$ désigne un radical de formule (V) tel que défini précédemment,
- . M' est choisi parmi Na, K, Li, $NH_4$.

Les composés de formule (IX) sont notamment décrits dans le brevet US-A-5 280 099.

De préférence, on utilise les tensioactifs siliconés comprenant au moins un groupement phosphate ou sulfate, et plus préférentiellement ceux à groupements phosphates, notamment les tensioactifs de formule (I).

L'émulsion utilisée selon l'invention comprend également, dans une phase grasse, au moins une première huile choisie parmi les huiles volatiles, les polyalkyl($C_1$-$C_{20}$)siloxanes et leurs mélanges, à une teneur d'au moins 65 % en poids par rapport au poids total de la phase grasse.

Par huile volatile, on entend dans la présente description, toute huile susceptible de s'évaporer au contact de la peau.

De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100 °C, et/ou dont la pression de vapeur, mesurée à $10^5$ Pa et à 25 °C, est supérieure

ou égale à 0,02 mm Hg (2,6 Pa) et/ou dont le point d'ébullition, mesuré à $10^5$ Pa est inférieur ou égal à 275 °C. L'huile volatile présente dans la phase grasse peut être choisie parmi les huiles volatiles hydrocarbonées, les huiles volatiles siliconées et leurs mélanges.

Parmi les huiles volatiles hydrocarbonées, on peut citer les isoparaffines et notamment l'isododécane.

Parmi les huiles siliconées volatiles, on peut citer :

- (i) les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- (ii) les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- (iii) les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane.

Les polyalkylsiloxanes selon l'invention sont à groupements terminaux triméthylsilyle. On peut utiliser de préférence ceux dont la viscosité à 25°C est inférieure ou égale à 0,06 $m^2/s$, parmi lesquels on peut citer :

- les polydiméthylsiloxanes linéaires et notamment ceux vendus sous les dénominations "DOW CORNING Fluid 200" par la société DOW CORNING
- les alkylméthylpolysiloxanes tels que la cétyldiméthicone (nom CTFA).

De préférence, la phase grasse comprend au moins 75% en poids, par rapport au poids total de ladite phase grasse, d'huile choisie parmi les huiles volatiles, siliconées et/ou hydrocarbonées, les polyalkylsiloxanes et leurs mélanges. Avantageusement, ladite phase grasse de l'émulsion comprend 100 % en poids, par rapport au poids total de la phase grasse, d'huiles volatiles, de polydiméthylsiloxanes ou de leurs mélanges.

La phase grasse de l'émulsion selon l'invention peut comprendre, en plus des premières huiles ci-dessus mentionnées, d'autres corps gras non volatils usuellement utilisés dans le domaine d'application envisagé. De préférence, la phase grasse comprend de 65 % à 98 % en poids, de préférence de 75 % à 98 %, par rapport au poids total de la phase grasse, d'huile choisie parmi les huiles volatiles, siliconées et/ou hydrocarbonées, les polyalkylsiloxanes et leurs mélanges et de 2 % à 35 % , de préférence de 2 % à 25 % en poids dudit autre corps gras.

Parmi les autres corps gras, on peut citer les huiles, les corps gras pâteux, les gommes et les cires végétales, minérales, animales et/ou synthétiques, ces derniers comprenant les corps gras siliconés.
On peut définir les composés gras pâteux à l'aide d'au moins une des propriétés physico-chimiques suivantes :

. une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
. un point de fusion de 25-70°C, de préférence 25-55°C.

Parmi les corps gras siliconés, on peut citer les huiles de silicone phénylées, ainsi que les gommes de silicones et les cires de silicone.
Parmi les corps gras non siliconés, on peut citer l'huile de paraffine, de vaseline, le perhydrosqualène, l'huile d'abricot, l'huile de germes de blé, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C; la cire d'abeilles; les cires végétales telles que la cire de Carnauba, de Candellila, d'Ouricury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

Les gommes de silicone peuvent répondre à la formule :

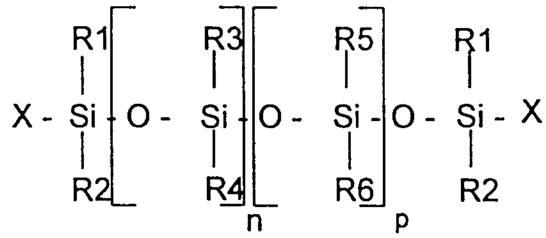

dans laquelle :

$R_1$, $R_2$, $R_5$ et $R_6$ sont, ensemble ou séparément, un radical alkyle ayant 1 à 6 atomes de carbone,
$R_3$ et $R_4$ sont, ensemble ou séparément, un radical alkyle ayant de 1 à 6 atomes de carbone, ou un radical aryle et notamment phényle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.

De manière générale, n et p peuvent 7prendre des valeurs de 0 à 5000, de préférence de 0 à 3000.
Comme gomme de silicone utilisable selon l'invention, on peut citer celles pour lesquelles :

. les substituants R1 à R6 et X représentent un groupement méthyle, p = 0 et n = 2700, comme celle vendue sous la dénomination SE30 par la société General Electric,
. les substituants R1 à R6 et X représentent un groupement méthyle, p = 0 et n = 2300, comme celle vendue sous la dénomination AK 500000 par la société Waker,
. les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
. les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
. les substituants R1, R2, R5, R6 et X représentent un groupement méthyle, les substituants R3 et R4 représentent un groupement phényle tel que le poids moléculaire du composé soit de 600 000, comme celle vendue sous les dénominations "761" ou "MIRASIL C-DPDM" par la société Rhône-Poulenc.

De manière préférentielle, on utilise comme autre corps gras les gommes de silicones.
Ces corps gras peuvent en particulier être choisis de manière variée par l'homme du métier afin de préparer une composition ayant les propriétés souhaitées, par exemple en consistance ou en texture. Ils sont de préférence utilisés à une teneur inférieure ou égale à 7 % en poids par rapport au poids total de l'émulsion, afin de conserver les propriétés avantageuse de l'émulsion utilisée selon l'invention.

La phase aqueuse de l'émulsion selon l'invention peut comprendre de l'eau ou une eau florale telle que l'eau de bleuet.
En outre, la phase aqueuse peut comprendre de 0 % à 14 % en poids, par rapport au poids total de la phase aqueuse, d'un monoalcool inférieur en $C_2$-$C_6$ et/ou d'un polyol tel que le glycérol, le butylèneglycol, l'isoprèneglycol, le propylèneglycol.

D'une manière générale, l'émulsion selon l'invention peut comprendre de 5 % à 40 % en poids de phase grasse, de préférence de 12 % à 30 %; de 0,5 % à 15 % en poids de tensioactif siliconé à groupement anionique, de préférence de 3 % à 6 %; et de 15 % à 94,5 % en poids de phase aqueuse, de préférence de 40 % à 70 %.

Par ailleurs, l'émulsion selon l'invention peut comprendre de 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

En outre, l'émulsion selon l'invention peut comprendre un ou plusieurs agents épaississants dans des concentrations préférentielles allant de 0 à 6 % en poids, par rapport au poids total de l'émulsion.
L'agent épaississant peut être choisi parmi :

- les biopolymères polysaccharidiques comme la gomme de xanthane, la gomme de caroube, la gomme de guar, les alginates, les celluloses modifiées telles que l'hydroxyéthylcellulose, la méthylcellulose,
- les polymères synthétiques comme les poly(méth)acrylates de glycéryle tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.

L'émulsion selon l'invention peut également comprendre une phase particulaire qui peut comprendre des pigments et/ou des nacres et/ou des charges habituellement utilisés dans les compositions cosmétiques.

Les pigments peuvent être présents dans l'émulsion à raison de 0-20 % en poids, par rapport au poids total de l'émulsion, et de préférence à raison de 2-15 %. Ils peuvent être blancs ou colorés, minéraux et/ou organiques. On peut citer, parmi les pigments et les nanopigments minéraux, les dioxydes de titane, de zirconium ou de cérium, ainsi que les oxydes de zinc, de fer ou de chrome, les nanotitanes, le bleu ferrique, les nacres telles que le mica recouvert d'oxyde de titane, d'oxyde de fer, de pigment naturel ou d'oxychlorure de bismuth ainsi que le mica titane coloré. Parmi les pigments organiques, on peut citer le noir de carbone, et les laques de baryum, strontium, calcium, aluminium.

Les charges, qui peuvent être présentes dans l'émulsion à raison de 0-20 % en poids, par rapport au poids total de l'émulsion, de préférence 0-10 %, peuvent être minérales ou de synthèse, lamellaires ou non lamellaires. On peut citer le talc, le mica, la silice, le kaolin, le Téflon, l'amidon, la nacre naturelle, le nitrure de bore, les microsphères telles que l'Expancel (Nobel Industrie), le polytrap (Dow Corning). De préférence, on utilise des charges sphériques dont la taille est inférieure à 25 μm telles que les poudres de polyéthylène, les poudres de Nylon, les microbilles de résine de silicone (Tospearls de Toshiba), les microsphères de silice, ces charges pouvant contribuer à améliorer les propriétés non transfert des émulsions de l'invention.

L'émulsion selon l'invention peut comprendre en outre un milieu cosmétiquement, pharmaceutiquement ou hygiéniquement acceptable. Elle peut comprendre alors tout additif usuellement utilisé dans le domaine cosmétique, pharmaceutique ou hygiénique, tels que des antioxydants, des colorants, des parfums, des huiles essentielles, des conservateurs, des actifs cosmétiques, des hydratants, des vitamines, des sphingolipides, des composés auto-bronzants tels que la DHA, des filtres solaires, des polymères liposolubles notamment hydrocarbonés, tels que le polybutène, les polyalkylènes, les polyacrylates et les polymères siliconés compatibles avec les corps gras. Bien entendu l'homme du métier veillera à choisir ce ou ses éventuels composés complémentaires, et/ou leur quantité, de manière telle que les propriétés avantageuses de la composition selon l'invention ne soient pas, ou substantiellement pas, altérées par l'adjonction envisagée. Ces additifs peuvent être présents dans la composition à raison de 0-10% en poids.

Les émulsions selon l'invention peuvent se présenter sous la forme d'un produit cosmétique et notamment sous la forme d'un produit de soin pour le corps et/ou le visage et/ou le cuir chevelu ou bien encore d'un produit de maquillage, en particulier un fond de teint, un fard à joues ou à paupières, un eye-liner, un mascara ou un rouge à lèvres.

Elles peuvent également se présenter sous forme non colorée, contenant éventuellement des actifs cosmétiques. L'émulsion selon l'invention peut être sous forme d'une crème, d'un lait ou d'un sérum, susceptible d'être utilisé comme produit de soin ou solaire.

Les exemples qui suivent servent à illustrer l'invention sans toutefois présenter un caractère limitatif.

**Exemples 1 à 7 :** Etude des propriétés de persistance des émulsions en fonction de la nature de la phase grasse.

On a réalisé 5 émulsions (fond de teint) conformes à l'invention (exemples 1 à 5) et 2 émulsions (fond de teint) ne faisant pas partie de l'invention (exemples 6 et 7), chaque émulsion étant différente par la nature de la phase grasse.

Les émulsions ont été préparées selon les compositions suivantes :

| | |
|---|---|
| - Phase grasse | X g |
| - Tensioactif siliconé à groupement phosphate (PECOSIL PS 100 de la société PHOENIX) | 5 g |
| - Coémulsionnant | 2 g |
| - Agents épaississants | 0,45 g |
| - Pigments | 7 g |

(suite)

| | |
|---|---|
| - Agents dispersants | 0,96 g |
| - Conservateurs | 0,6 g |
| - Eau | qsp 100 g |

On a reporté dans le tableau I la constitution de la phase grasse de chaque émulsion.

Dans le tableau I :

- (I) désigne un exemple selon l'invention
- (HI) désigne un exemple ne faisant pas partie de l'invention
- la cyclo D6 est la cyclohexadiméthylsiloxane

On a préparé les compositions, de manière usuelle, en chauffant à 65 °C les ingrédients de la phase grasse, le coémulsionnant et une partie des conservateurs. Puis on a préparé la phase aqueuse en mélangeant l'eau, le tensioactif siliconé et les pigments préalablement dispersés avec les agents dispersants, tout en chauffant à 80 °C. Ensuite, la phase grasse a été versée dans la phase aqueuse à 65 °C sous agitation à l'aide d'une turbine puis on a ajouté à l'émulsion obtenue les agents épaississants et le reste des conservateurs à 40 °C.

On a ensuite déterminé les propriétés de persistance de ces émulsions. Pour cela, on a appliqué 0,05 g de chaque émulsion sur une surface de 50 $cm^2$ sur l'avant bras puis on a laissé sécher la composition appliquée pendant 5 minutes. On a ensuite appliqué une bande de tissu en polyester sur la partie de l'avant bras traité. Ensuite, à l'aide d'un appareil, on a animé la bande d'un mouvement de translation vertical, au contact de l'avant-bras traité. Le tissu a été maintenu tendu à l'aide d'un contrepoids créant ainsi un frottement du tissu pendant la translation. On a réalisé 10 mouvements aller et retour de frottement.

On a ensuite noté les traces colorées qui se sont éventuellement déposées sur le tissu selon la notation suivante :

tissu très taché : note = 0
tissu sans trace : note = 10

On considère qu'un fond de teint transfère peu lorsque la notation est égale ou supérieure à 7,5.

Les résultats obtenus sont reportés dans le tableau I.

TABLEAU I

| EXEMPLES | 1 (I) | 2 (I) | 3 (I) | 4 (I) | 5 (I) | 6 (HI) | 7 (HI) |
|---|---|---|---|---|---|---|---|
| Huile H | PDMS de viscosité $10^{-5}$ $m^2/s$ | | Cyclohexadiméthylsiloxane | Isododécane | Huile d'abricot | | |
| Teneur en huile H par rapport à la phase grasse | 100 % | 25 % | 100 % | 25 % | 25 % | 50 % | 100 % |
| Teneur en cyclo D6 par rapport à la phase grasse | 0 % | 75 % | 0 % | 75 % | 75 % | 50 % | 0 % |
| Teneur en huile H par rapport à l'émulsion | 20 % | 7,5 % | 40 % | 5 % | 5 % | 9 % | 19 % |
| Poids total (X) de la phase grasse dans l'émulsion | 20 g | 30 g | 40 g | 20 g | 20 g | 18 g | 19 g |
| Résultat non transfert | 8 | 8 | 8,5 | 7,5 | 7,5 | 5,5 | 5 |

Les résultats obtenus montrent que les émulsions dont la phase grasse ne comprend que la PDMS ou que la silicone volatile (exemples 1 et 3) présentent de bonnes propriétés de persistance et ne transfèrent pas sur le tissu.

De même, les émulsions dont la phase grasse comprend un mélange d'huile siliconée volatile avec un PDMS (exemple 2) ou avec une huile volatile hydrocarbonée (exemple 4) ne transfèrent pas sur le tissu.

Par contre, parmi les émulsions comprenant un mélange d'huile siliconée volatile et d'huile d'abricot (exemples 5 à 7), seule celle dont la teneur en huile d'abricot est égale à 5 % en poids par rapport au poids total de l'émulsion (exemple 5) présente de bonnes propriétés de persistance. Ces exemples montrent donc que les huiles autres que les PDMS et les huiles volatiles doivent être présentes dans la phase grasse de l'émulsion à des teneurs faibles.

**EXEMPLE 8 :**

On a préparé un fond de teint ayant la composition suivante :

| | |
|---|---|
| - Cyclohexadimethylsiloxane | 14 g |
| - Monostéarate de sorbitane oxyéthyléné à 20 moles d'oxyde d'éthylène | 2 g |
| - Pigments | 7 g |
| - Agents dispersants | 1 g |
| - Agents épaississants | 0,5 g |
| - Tensioactif siliconé à groupement phosphate (PECOSIL PS100) | 5 g |
| - Glycérine | 5 g |
| - Conservateurs | 0,6 g |
| - Eau déminéralisée stérilisée q.s.p. | 100 g |

La composition est préparée selon le même mode opératoire des exemples 1 à 7. Le fond de teint ainsi obtenu s'étale facilement sur le visage et présente après application une bonne tenue et une bonne persistance.

**EXEMPLE 9 :**

On a préparé un fond de teint ayant la composition suivante :

| | |
|---|---|
| - Cyclohexa dimethylsiloxane | 15 g |
| - Mono-stéarate de sorbitane oxyéthyléné à 20 moles d'oxyde d'éthylène | 2 g |
| - Pigments | 7 g |
| - Agents dispersants | 1 g |
| - Agents épaississants | 0,5 g |
| - Tensioactif siliconé à groupement sulfate vendu sous la dénomination WATER SOLUBLE SULFATE par la Société SILTECH | 14,3 g |
| - Glycérine | 5 g |
| - Conservateurs | 0,6 g |
| - Eau déminéralisée stérilisée q.s.p. | 100 g |

La composition est préparée selon le même mode opératoire des exemples 1 à 7. On obtient ainsi un fond de teint qui s'étale facilement sur la peau sans sensation de gras et ne transfère pas au contact d'un tissu.

**EXEMPLE 10 :**

On a préparé un fond de teint ayant la composition suivante :

| | |
|---|---|
| - Cyclohexa diméthylsiloxane | 15 g |
| - Poly dimethylsiloxane (viscosité $10^{-5}$ $m^2/s$) | 15 g |
| - Mono-stéarate de sorbitane oxyéthylénée à 20 moles d'oxyde d'éthylène | 2 g |
| - Pigments | 7 g |
| - Agents dispersants | 1 g |
| - Agents épaississants | 0,5 g |
| - Tensioactif siliconé à groupement phosphate (PECOSIL PS 100) | 5 g |
| - Glycérine | 5 g |
| - Conservateurs | 0,6 g |
| - Eau déminéralisée stérilisée q.s.p. | 100 g |

La composition est préparée selon le même mode opératoire des exemples 1 à 7. Le fond de teint ainsi obtenu présente de bonnes propriétés cosmétiques et ne transfère pas sur un tissu après application sur la peau.

**EXEMPLE 11 :**

On a préparé une crème ayant la composition suivante :

| | |
|---|---|
| - Cyclohexadiméthylsiloxane | 20 g |
| - Monostéarate de sorbitane oxyéthylène à 20 moles d'oxyde d'éthyléne | 2 g |
| - Agents dispersants | 1 g |
| - Agents épaississants | 0,5 g |
| - Tensioactif siliconé à groupement phosphate (PECOSIL PS 100) | 5 g |
| -Glycérine | 5 g |
| - Conservateurs | 0,6 g |
| - Eau déminéralisée stérilisée q.s.p. | 100 g |

La composition est préparée selon le même mode opératoire des exemples 1 à 7. On obtient une crème qui s'étale facilement sur la peau et qui ne transfère pas sur un tissu appliqué sur la peau.

**Exemple 12 :**

On a préparé la composition suivante :

| | |
|---|---|
| - cyclohexadiméthylsiloxane | 11 g |
| - mélange de polydiphényl diméthyl siloxane et de cyclopentadiméthylsiloxane (15/85) ("MIRASIL C-DPDM" de Rhône-Poulenc) | 3 g |

(suite)

| | |
|---|---|
| - épaississants | 1,23 g |
| - poudre de nylon | 2 g |
| - pigments | 8 g |
| - tensioactif siliconé à groupement phosphate ("PECOSIL PS 100") | 5 g |
| - monostéarate de sorbitane oxyéthyléné à 20 moles d'oxyde d'éthylène | 2 g |
| - glycérine | 5 g |
| - conservateurs qs | |
| - eau déminéralisée qsp | 100 g |

La composition est préparée selon le même mode opératoire des exemples 1 à 7. On obtient un fond-de-teint qui s'étale facilement sur la peau et qui ne transfère pas sur un tissu appliqué sur la peau.

**Revendications**

1. Utilisation cosmétique de l'association d'un tensioactif siliconé comprenant au moins un groupement anionique et d'une phase grasse comprenant première huile choisie parmi les huiles volatiles, les polyalkyl($C_1$-$C_{20}$)siloxanes et leurs mélanges, dans une émulsion huile-dans-eau pour diminuer le transfert et/ou la migration et/ou pour améliorer la persistance et/ou la tenue de ladite émulsion ou d'une composition la comprenant, sur la peau.

2. Utilisation de l'association d'un tensioactif siliconé comprenant au moins un groupement anionique et d'une phase grasse comprenant première huile choisie parmi les huiles volatiles, les polyalkyl($C_1$-$C_{20}$)siloxanes et leurs mélanges, pour la préparation d'une émulsion huile-dans-eau pour diminuer le transfert et/ou la migration et/ou pour améliorer la persistance et/ou la tenue de ladite émulsion ou d'une composition la comprenant, sur la peau.

3. Utilisation cosmétique de l'association d'un tensioactif siliconé comprenant au moins un groupement anionique et d'une première huile choisie parmi les huiles volatiles, les polyalkyl($C_1$-$C_{20}$)siloxanes et leurs mélanges, dans une émulsion huile-dans-eau qui ne transfère pas et/ou qui ne migre pas sur la peau.

4. Utilisation selon l'une quelconque des revendications précédentes, caractérisée par le fait que le tensioactif siliconé à groupement anionique est choisi parmi les tensioactifs à groupement phosphate, sulfate, sulfonate et/ou carboxylate.

5. Utilisation selon l'une quelconque des revendications précédentes, caractérisée par le fait que le tensioactif siliconé à groupement phosphate est choisi parmi ceux de formule (I) :

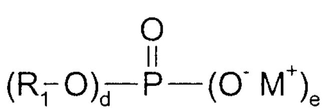

(I)

dans laquelle $R_1$ désigne le radical de formule (V) suivante :

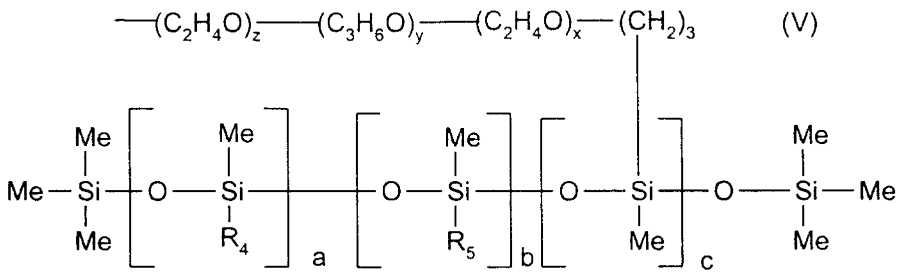

(V)

formules dans lesquelles :

Me désigne le radical méthyle,
$C_2H_4O$ représente le groupement $-CH_2-CH_2-O-$,
$C_3H_6O$ représente le groupement $-CH_2-CH(CH_3)-O-$
a est un nombre entier allant de 0 à 200,
b est un nombre entier allant de 0 à 200,
c est un nombre entier allant de 1 à 200,
$R_4$ désigne un radical $-(CH_2)_nCH_3$ ou phényle, n étant un nombre entier allant de 0 à 10,
$R_5$ désigne le radical $-(CH_2)_3-(OCH_2CH_2)_x-(OCH_2CH(CH_3))_y-(OCH_2CH_2)_z-OH$,
x, y, z sont des nombres entiers allant indépendamment de 0 à 20,
d et e vont de 1 à 2, avec d+e=3 ,
M est choisi dans le groupe constitué de H, Na, K, Li, $NH_4$ et $N(CH_2CH_2OH)_3$.

**6.** Utilisation selon l'une quelconque des revendications 1 à 4, caractérisée par le fait que le tensioactif siliconé à groupement phosphate est choisi parmi ceux de formule (II) :

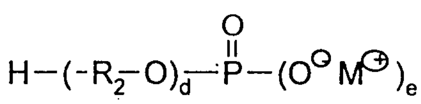

(II)

dans laquelle $R_2$ désigne le radical de formule (VI) suivante :

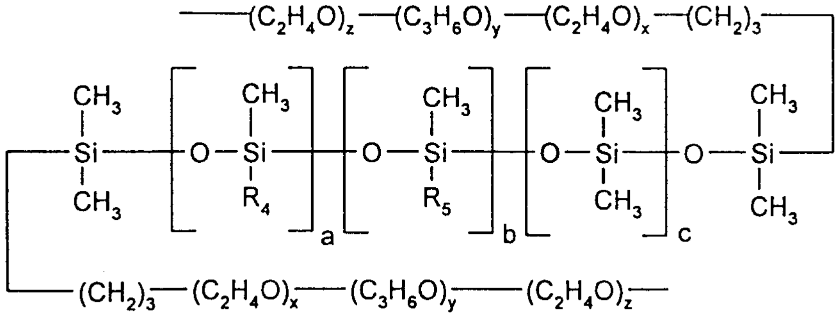

(VI)

formules dans lesquelles :

Me désigne le radical méthyle,
$C_2H_4O$ représente le groupement $-CH_2-CH_2-O-$,
$C_3H_6O$ représente le groupement $-CH_2-CH(CH_3)-O-$
a est un nombre entier allant de 0 à 200,
b est un nombre entier allant de 0 à 200,
c est un nombre entier allant de 1 à 200,
$R_4$ désigne un radical $-(CH_2)_nCH_3$ ou phényle, n étant un nombre entier allant de 0 à 10,
$R_5$ désigne le radical $-(CH_2)_3-(OCH_2CH_2)_x-(OCH_2CH(CH_3))_y-(OCH_2CH_2)_z-OH$,

x, y, z sont des nombres entiers allant indépendamment de 0 à 20,
d et e vont de 1 à 2 sous réserve que d+e=3 ,
M est choisi dans le groupe constitué de H, Na, K, Li, $NH_4$ et $N(CH_2CH_2OH)_3$.

**7.** Utilisation selon l'une quelconque des revendications 1 à 4, caractérisée par le fait que le tensioactif siliconé à groupement phosphate est choisi parmi ceux de formule (III) :

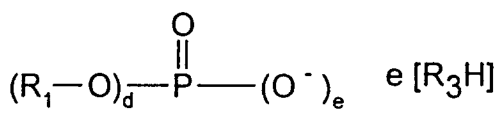

(III)

dans laquelle $R_1$ est le radical de formule (V) suivante :

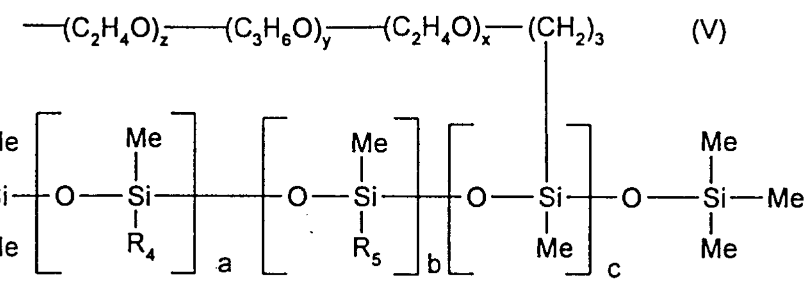

(V)

formules dans lesquelles :

Me désigne le radical méthyle,
$C_2H_4O$ représente le groupement $-CH_2-CH_2-O-$,
$C_3H_6O$ représente le groupement $-CH_2-CH(CH_3)-O-$
a est un nombre entier allant de 0 à 200,
b est un nombre entier allant de 0 à 200,
c est un nombre entier allant de 1 à 200,
$R_4$ désigne un radical $-(CH_2)_nCH_3$ ou phényle, n étant un nombre entier allant de 0 à 10,
$R_5$ désigne le radical $-(CH_2)_3-(OCH_2CH_2)_x-(OCH_2CH(CH_3))_y-(OCH_2CH_2)_z-OH$,
x, y, z sont des nombres entiers allant indépendamment de 0 à 20,
d et e vont de 1 à 2 sous réserve que d+e=3 ,
$R_3$ est choisi parmi :

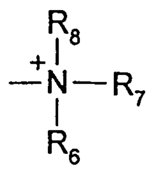

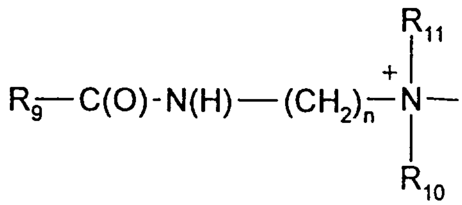

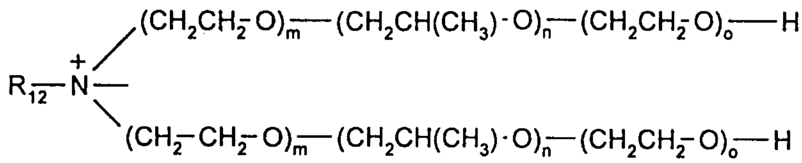

et

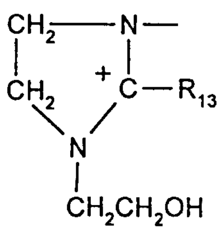

formules dans lesquelles :

$R_6$ à $R_9$ désignent, indépendamment, un radical alkyle ayant de 1 à 20 atomes de carbone,
$R_{10}$ et $R_{11}$ désignent, indépendamment, un radical alkyle ayant de 1 à 3 atomes de carbone,
$R_{12}$ et $R_{13}$ désignent, indépendamment, un radical alkyle ayant de 6 à 20 atomes de carbone,
m, n et o désignent, indépendamment un nombre entier allant de 0 à 20.

**8.** Utilisation selon l'une quelconque des revendications 1 à 4, caractérisée par le fait que le tensioactif siliconé à groupement phosphate est choisi parmi ceux de formule (IV) :

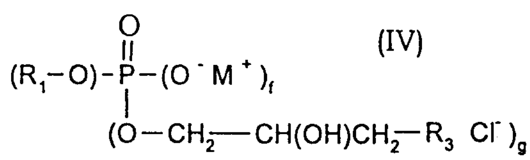

(IV)

dans laquelle $R_1$ est le radical de formule (V) suivante :

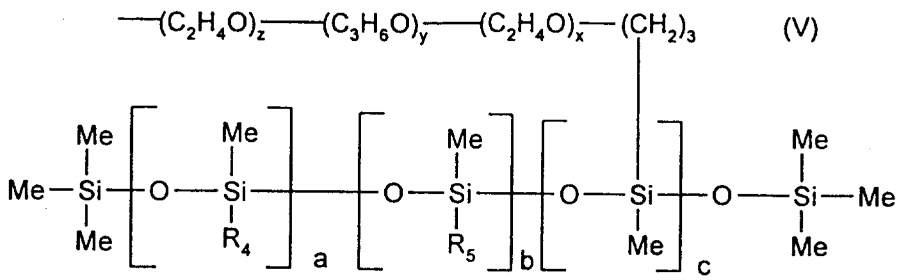

(V)

formules dans lesquelles :

Me désigne le radical méthyle,
$C_2H_4O$ représente le groupement $-CH_2-CH_2-O-$,
$C_3H_6O$ représente le groupement $-CH_2-CH(CH_3)-O-$
a est un nombre entier allant de 0 à 200,
b est un nombre entier allant de 0 à 200,
c est un nombre entier allant de 1 à 200,
$R_4$ désigne un radical $-(CH_2)_nCH_3$ ou phényle, n étant un nombre entier allant de 0 à 10,
$R_5$ désigne le radical $-(CH_2)_3-(OCH_2CH_2)_x-(OCH_2CH(CH_3))_y-(OCH_2CH_2)_z-OH$,
x, y, z sont des nombres entiers allant indépendamment de 0 à 20,
f est égal à 0 ou 1, g est égal à, 1 ou 2 et f+g=2 ,
$R_3$ est choisi parmi :

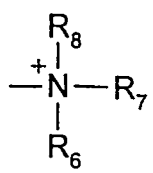

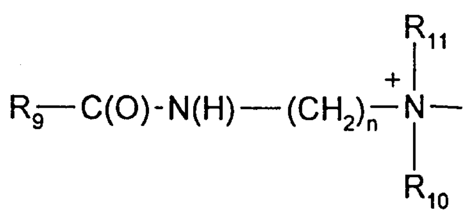

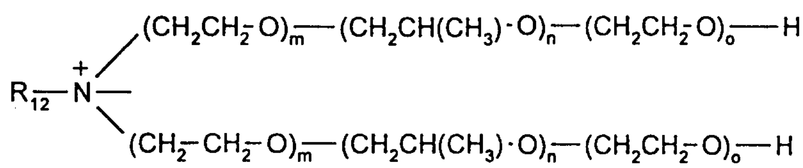

et

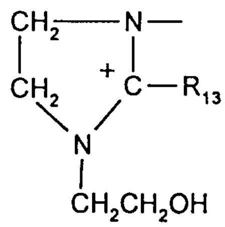

formules dans lesquelles :

$R_6$ à $R_9$ désignent, indépendamment, un radical alkyle ayant de 1 à 20 atomes de carbone,
$R_{10}$ et $R_{11}$ désignent, indépendamment, un radical alkyle ayant de 1 à 3 atomes de carbone,
$R_{12}$ et $R_{13}$ désignent, indépendamment, un radical alkyle ayant de 6 à 20 atomes de carbone,
m, n et o désignent, indépendamment un nombre entier allant de 0 à 20.

**9.** Utilisation selon l'une quelconque des revendications 1 à 4, caractérisée par le fait que le tensioactif siliconé à groupement sulfate est choisi parmi ceux de formule (VII) :

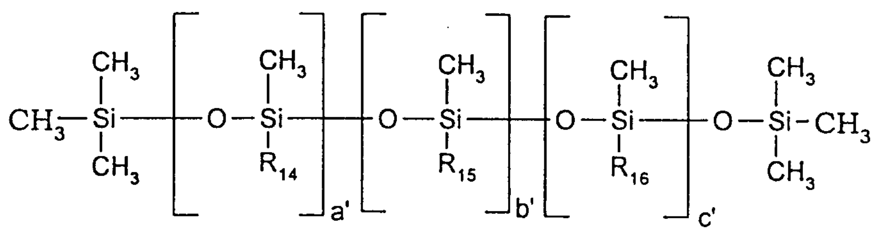 (VII)

dans laquelle

$R_{14}$ désigne un radical alkyl ayant de 1 à 8 atomes de carbone ou un radical phényle,
$R_{15}$ désigne un radical
$-(CH_2)_3-O-(CH_2CH_2O)_u-(CH_2CH(CH_3)-O)_v-(CH_2CH_2O)_w-SO_3^- M^+$,
M est choisi parmi Na, K, Li, $NH_4$,
$R_{16}$ désigne un radical
$-(CH_2)_3-O-(CH_2CH_2O)_u-(CH_2CH(CH_3)-O)_v-(CH_2CH_2O)_w-H$,
dans lequel u, v, w désignent, indépendamment, un nombre entier allant de 0 à 100,
a' et c' désignent, indépendamment, un nombre entier allant de 0 à 50,
b' désigne un nombre entier allant de 1 à 50.

**10.** Utilisation selon l'une quelconque des revendications 1 à 4, caractérisée par le fait que le tensioactif siliconé à groupement sulfonate est choisi parmi ceux obtenus par la réaction d'une silicone de formule (VIII) :

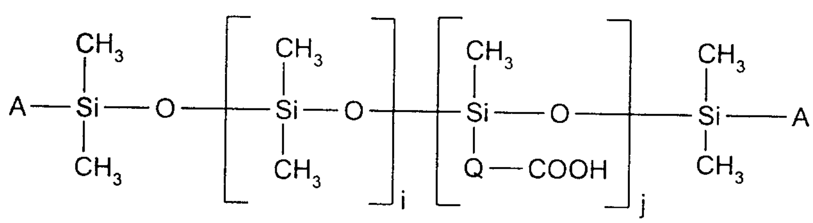 (VIII)

dans laquelle

Q désigne $(CH_2)_r$, r étant un nombre entier allant de 3 à 17,
j désigne soit un nombre entier allant de 1 à 10 et A désigne un radical méthyle,
soit j= 0 et A désigne un radical -Q-COOH,

i désigne un nombre entier allant de 1 à 200,
avec un dérivé de taurine de formule $R_{17}$-NH-$(CH_2)_2$-$SO_3M$ ,
dans laquelle $R_{17}$ désigne un radical alkyle ayant de 1 à 40 atomes de carbone, et
M est choisi parmi Na, K, Li, $NH_4$.

**11.** Utilisation selon l'une quelconque des revendications 1 à 4, caractérisée par le fait que le tensioactif siliconé à groupement sulfonate est choisi parmi ceux de formule (IX) :

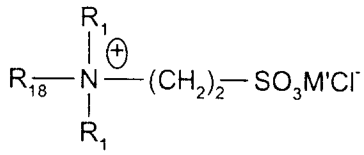

(IX)

dans laquelle

$R_{18}$ désigne un radical alkyle ayant de 1 à 40 atomes de carbone,
$R_1$ désigne le radical de formule (V) suivante :

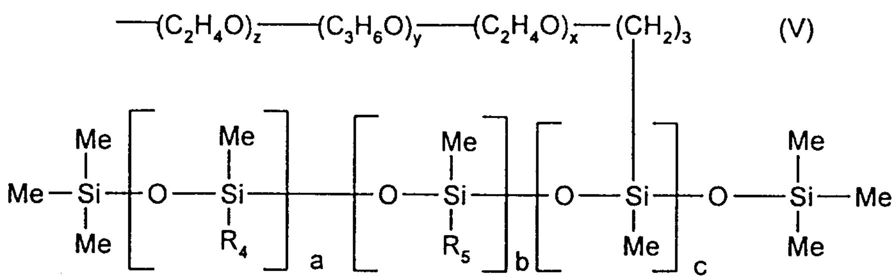

(V)

formules dans lesquelles :
Me désigne le radical méthyle,
$C_2H_4O$ représente le groupement $-CH_2-CH_2-O-$,
$C_3H_6O$ représente le groupement $-CH_2-CH(CH_3)-O-$
a est un nombre entier allant de 0 à 200,
b est un nombre entier allant de 0 à 200,
c est un nombre entier allant de 1 à 200,
$R_4$ désigne un radical $-(CH_2)_nCH_3$ ou phényle, n étant un nombre entier allant de 0 à 10,
$R_5$ désigne le radical $-(CH_2)_3-(OCH_2CH_2)_x-(OCH_2CH(CH_3))_y-(OCH_2CH_2)_z-OH$,
x, y, z sont des nombres entiers allant indépendamment de 0 à 20,
M' est choisi parmi Na, K, Li, $NH_4$.

**12.** Utilisation selon l'une quelconque des revendications précédentes, caractérisée par le fait que ladite première huile est présente à raison d'au moins 65 % en poids par rapport au poids total de la phase grasse de l'émulsion.

**13.** Utilisation selon l'une quelconque des revendications précédentes, caractérisée par le fait que l'huile volatile est choisie parmi les huiles volatiles hydrocarbonées, les huiles volatiles siliconées et leurs mélanges.

**14.** Utilisation selon l'une quelconque des revendications précédentes, caractérisée par le fait que l'huile volatile est choisie parmi les isoparaffines, les silicones volatiles cycliques ayant de 3 à 8 atomes de silicium et de préférence de 4 à 6, les cyclocopolymères du type diméthylsiloxane/méthylalkylsiloxane, les silicones volatiles linéaires ayant de 2 à 9 atomes de silicium, et leurs mélanges.

**15.** Utilisation selon l'une quelconque des revendications précédentes, caractérisée par le fait que l'huile volatile est choisie parmi l'isododécane, la cyclotétradiméthylsiloxane, la cyclopentadiméthylsiloxane, la cyclohexadiméthylsiloxane, le cyclocopolymère diméthylsiloxane/méthyloctylsiloxane, l'hexaméthyldisiloxane, l'hexyl heptaméthyltrisiloxane, l'octyl heptaméthyltrisiloxane, et leurs mélanges.

**16.** Utilisation selon l'une quelconque des revendications précédentes, caractérisée par le fait que les polyalkyl($C_1$-$C_{20}$)siloxanes ont une viscosité à 25 °C inférieure ou égale à 0,06 $m^2$/s.

**17.** Utilisation selon l'une quelconque des revendications précédentes, caractérisée par le fait que les polyalkyl($C_1$-$C_{20}$)siloxanes sont choisis parmi les polydiméthylsiloxanes linéaires, les alkylméthylpolysiloxanes et leurs mélanges.

**18.** Utilisation selon l'une des revendications 1 à 17, caractérisée par le fait que la phase grasse comprend, en outre, au moins un autre corps gras, autre que ladite première huile, à une teneur inférieure ou égale à 7 % en poids par rapport au poids total de l'émulsion.

**19.** Utilisation selon la revendication 18, caractérisée par le fait que la phase grasse comprend de 65 % à 98 % en poids, par rapport au poids total de la phase grasse, de ladite première huile, et de 2 % à 35 % en poids dudit autre corps gras.

**20.** Utilisation selon l'une des revendications 18 et 19, caractérisée par le fait que ledit autre corps gras est choisi parmi les huiles, les corps gras pâteux, les gommes et/ou les cires végétales, minérales, animales et/ou synthétiques.

**21.** Utilisation selon l'une quelconque des revendications précédentes, caractérisée par le fait que la phase grasse comprend au moins 75 % en poids, par rapport au poids total de la phase grasse, d'huile choisie parmi les huiles volatiles, les polyalkyl($C_1$-$C_{20}$)siloxanes et leurs mélanges.

**22.** Utilisation selon l'une quelconque des revendications 1 à 17, caractérisée par le fait que la phase grasse comprend 100 % en poids, par rapport au poids total de la phase grasse, de ladite première huile.

**23.** Utilisation selon l'une quelconque des revendications précédentes, caractérisée par le fait que l'émulsion comprend, par rapport au poids total de l'émulsion, de 5 % à 40 % en poids de phase grasse, de 0,5 % à 15 % en poids de tensioactif siliconé à groupement anionique et de 15 % à 94,5 % en poids de phase aqueuse.

**24.** Utilisation selon l'une quelconque des revendications précédentes, caractérisée par le fait que l'émulsion comprend, par rapport au poids total de l'émulsion, de 12 % à 30 % en poids de phase grasse, de 3 % à 6 % en poids de tensioactif siliconé à groupement anionique, et de 40 % à 70 % en poids de phase aqueuse.

**25.** Utilisation selon l'une quelconque des revendications précédentes, caractérisée par le fait que l'émulsion est comprise dans une composition cosmétique.

**26.** Utilisation selon la revendication 2 caractérisée par le fait que l'émulsion est comprise dans une composition cosmétique, pharmaceutique ou hygiénique.

**27.** Utilisation selon la revendication 25 ou 26 caractérisée par le fait que la composition se présente sous forme d'un produit de maquillage tel qu'un fond de teint, un fard à joues, un fard à paupières, un eye-liner, un mascara ou un rouge à lèvres ou se présente sous forme d'une crème, d'un lait ou d'un sérum, susceptible d'être utilisée comme produit de soin ou comme produit solaire.

**28.** Emulsion huile-dans-eau comprenant :

- (a) une phase aqueuse,
- (b) une phase grasse comprenant au moins une première huile choisie parmi les huiles volatiles, les polyalkyl($C_1$-$C_{20}$)siloxa-nes, et leurs mélanges, à une teneur d'au moins 65 % en poids par rapport au poids total de la phase grasse,
- (c) un tensioactif siliconé comprenant au moins un groupement anionique choisi parmi :
- (i) les tensioactifs siliconés à groupement phosphate de formule (I), (II), (III) ou (IV) :

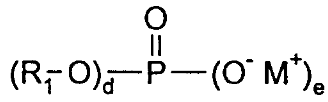
(I)

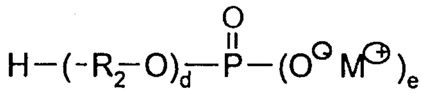
(II)

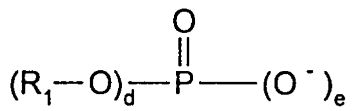
(III)

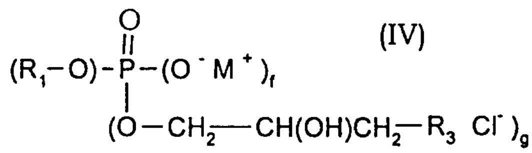
(IV)

formules dans lesquelles :

$R_1$ désigne le radical de formule (V) suivante :

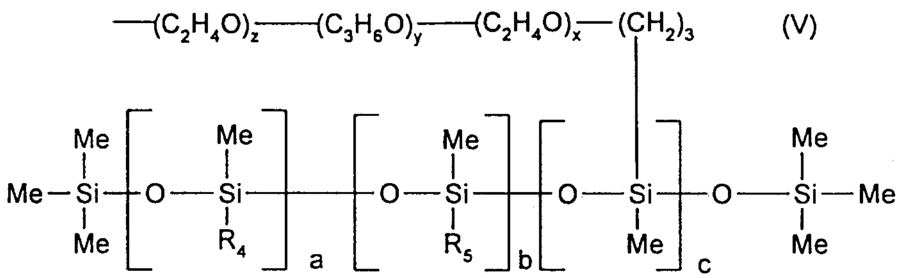
(V)

$R_2$ désigne le radical de formule (VI) suivante :

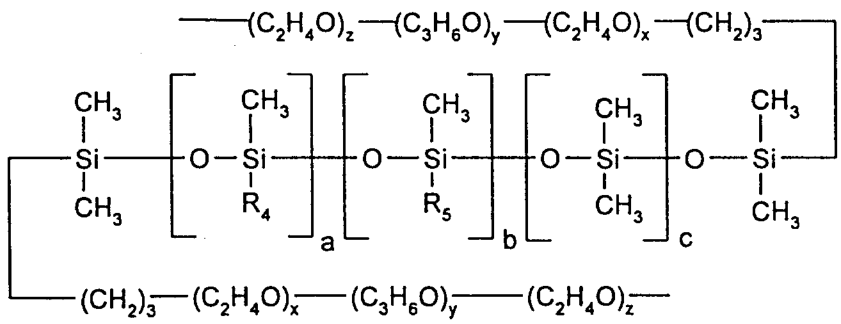
(VI)

formule dans laquelle :
Me désigne le radical méthyle,
$C_2H_4O$ représente le groupement $-CH_2-CH_2-O-$,
$C_3H_6O$ représente le groupement $-CH_2-CH(CH_3)-O-$
a est un nombre entier allant de 0 à 200,
b est un nombre entier allant de 0 à 200,
c est un nombre entier allant de 1 à 200,
$R_4$ désigne un radical $-(CH_2)_nCH_3$ ou phényle, n étant un nombre entier allant de 0 à 10,

$R_5$ désigne le radical $-(CH_2)_3-(OCH_2CH_2)_x-(OCH_2CH(CH_3))_y-(OCH_2CH_2)_z-OH$,
x, y, z sont des nombres entiers allant indépendamment de 0 à 20,
$R_3$ est choisi parmi :

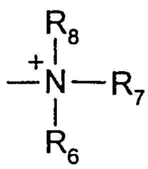

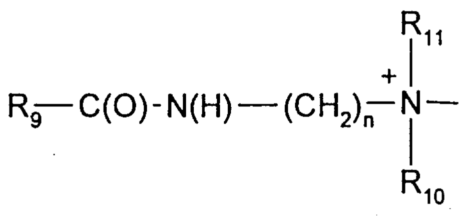

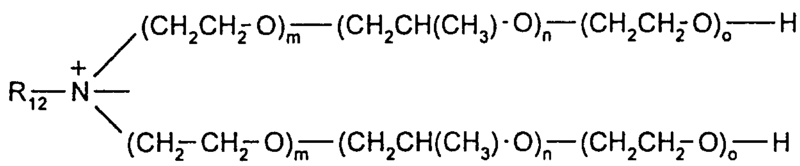

et

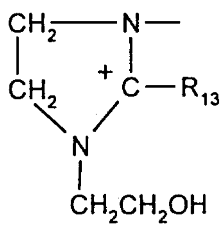

formules dans lesquelles :
$R_6$ à $R_9$ désignent, indépendamment, un radical alkyle ayant de 1 à 20 atomes de carbone,
$R_{10}$ et $R_{11}$ désignent, indépendamment, un radical alkyle ayant de 1 à 3 atomes de carbone,
$R_{12}$ et $R_{13}$ désignent, indépendamment, un radical alkyle ayant de 6 à 20 atomes de carbone,
m, n et o désignent, indépendamment un nombre entier allant de 0 à 20,
d et e vont de 1 à 2, avec d+e=3 ,
f est égal à 0 ou 1, g est égal à 1 ou 2 et f+g=2 ,
M est choisi dans le groupe constitué de H, Na, K, Li, $NH_4$ et $N(CH_2CH_2OH)_3$,
(ii) les tensioactifs siliconés à groupement sulfate de formule (VII) :

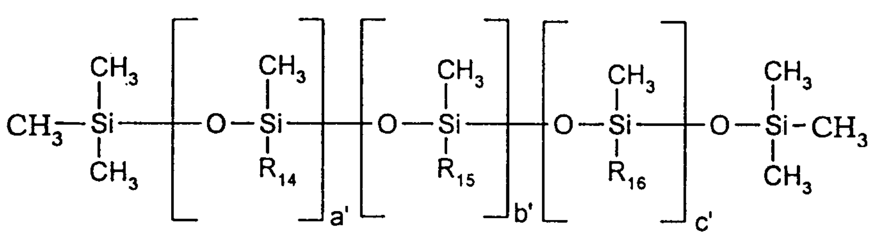

(VII)

dans laquelle
$R_{14}$ désigne un radical alkyl ayant de 1 à 8 atomes de carbone ou un radical phényle,
$R_{15}$ désigne un radical
$-(CH_2)_3-O-(CH_2CH_2O)_u-(CH_2CH(CH_3)-O)_v-(CH_2CH_2O)_w-SO_3^-\ M'^+$,
M' est choisi parmi Na, K, Li, $NH_4$,
$R_{16}$ désigne un radical

$-(CH_2)_3-O-(CH_2CH_2O)_u-(CH_2CH(CH_3)-O)_v-(CH_2CH_2O)_w-H$,
dans lequel u, v, w désignent, indépendamment, un nombre entier allant de 0 à 100,
a' et c' désignent, indépendamment, un nombre entier allant de 0 à 50,
b' désigne un nombre entier allant de 1 à 50,

- (iii) les tensioactifs siliconés à groupement sulfonate obtenus par la réaction d'une silicone de formule (VIII) :

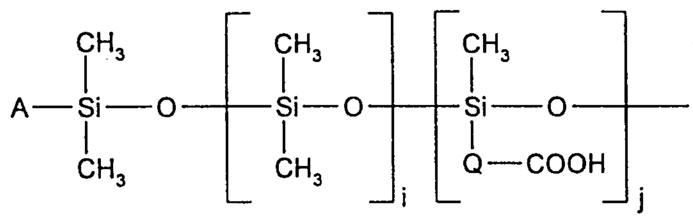

(VIII)

dans laquelle

Q désigne $(CH_2)_r$, r étant un nombre entier allant de 3 à 17,
j désigne soit un nombre entier allant de 1 à 10 et A désigne un radical méthyle,
soit j= 0 et A désigne un radical -Q-COOH,
i désigne un nombre entier allant de 1 à 200,
avec un dérivé de taurine de formule $R_{17}-NH-(CH_2)_2-SO_3M''$,
dans laquelle $R_{17}$ désigne un radical alkyle ayant de 1 à 40 atomes de carbone, et
M" est choisi parmi Na, K, Li, $NH_4$,

- (iv) les tensioactifs siliconés à groupement sulfonate de formule (IX) :

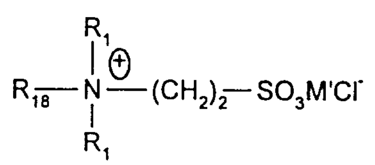

(IX)

dans laquelle

$R_{18}$ désigne un radical alkyle ayant de 1 à 40 atomes de carbone,
$R_1$ désigne le radical de formule (V) tel que défini pour le composé de formule (I),
M' est choisi parmi Na, K, Li, $NH_4$,
sous réserve que lorsque le tensioactif siliconé est de formule (I), la phase grasse de l'émulsion comprend un autre corps gras, autre que les huiles volatiles et les polyalkyl($C_1$-$C_{20}$)siloxanes, à une teneur inférieure ou égale à 7 % en poids par rapport au poids total de l'émulsion.

**29.** Emulsion selon la revendication 28 caractérisée par le fait que l'huile volatile est choisie parmi les huiles volatiles hydrocarbonées, les huiles volatiles siliconées et leurs mélanges.

**30.** Emulsion selon l'une des revendications 28 et 29 caractérisée par le fait que l'huile volatile est choisie parmi les isoparaffines, les silicones volatiles cycliques ayant de 3 à 8 atomes de silicium et de préférence de 4 à 6, les cyclocopolymères du type diméthylsiloxane/méthylakylsiloxane, les silicones volatiles linéaires ayant de 2 à 9 atomes de silicium, et leurs mélanges.

**31.** Emulsion selon l'une des revendications 28 à 30, caractérisée par le fait que l'huile volatile est choisie parmi l'isododécane, la cyclotétradiméthylsiloxane, la cyclopentadiméthylsiloxane, la cyclohexadiméthylsiloxane, le cyclocopolymère diméthylsiloxane/méthyloctylsiloxane, l'hexaméthyldisiloxane, l'hexyl heptaméthyltrisiloxane, l'octyl heptaméthyltrisiloxane, et leurs mélanges.

**32.** Emulsion selon l'une quelconque des revendications 28 à 31, caractérisée par le fait que les polyalkyl($C_1$-$C_{20}$)siloxanes ont une viscosité à 25 °C inférieure ou égale à 0,06 $m^2$/s.

**33.** Emulsion selon l'une quelconque des revendications 28 à 32, caractérisée par le fait que les polyalkyl($C_1$-$C_{20}$)siloxanes sont choisis parmi les polydiméthylsiloxanes linéaires, les alkylméthylpolysiloxanes et leurs mélanges.

**34.** Emulsion selon l'une quelconque des revendications 28 à 33, caractérisée par le fait que la phase grasse comprend au moins 75 % en poids, par rapport au poids total de la phase grasse, d'huile choisie parmi les huiles volatiles, les polyalkyl($C_1$-$C_{20}$)siloxanes et leurs mélanges.

**35.** Emulsion selon l'une quelconques des revendications 28 à 34, caractérisée par le fait que la phase grasse comprend 100 % en poids, par rapport au poids total de la phase grasse, de ladite première huile, lorsque ledit tensioactif siliconé est différent de ceux de formule (I).

**36.** Emulsion selon l'une des revendications 28 à 35, caractérisée par le fait que la phase grasse comprend, en outre, au moins un autre corps gras, autre que ladite première huile, à une teneur inférieure ou égale à 7 % en poids par rapport au poids total de l'émulsion.

**37.** Emulsion selon l'une des revendications 28 à 36, caractérisée par le fait que ledit autre corps gras est choisi parmi les huiles, les corps gras pâteux, les gommes et/ou les cires végétales, minérales, animales et/ou synthétiques.

**38.** Emulsion selon l'une des revendications 28 à 37, caractérisée par le fait qu'elle comprend, par rapport au poids total de l'émulsion, de 5 % à 40 % en poids de phase grasse, de 0,5 % à 15 % en poids de tensioactif siliconé à groupement anionique et de 15 % à 94,5 % en poids de phase aqueuse.

**39.** Emulsion selon l'une des revendications 28 à 37, caractérisée par le fait qu'elle comprend, par rapport au poids total de l'émulsion, de 12 % à 30 % en poids de phase grasse, de 3 % à 6 % en poids de tensioactif siliconé à groupement anionique, et de 40 % à 70 % en poids de phase aqueuse.

**40.** Composition cosmétique, pharmaceutique ou hygiénique, caractérisée par le fait qu'elle comprend une émulsion selon l'une des revendications 28 à 39.

**41.** Composition selon la revendication 40, caractérisée par le fait qu'elle se présente sous forme d'un produit de maquillage tel qu'un fond de teint, un fard à joues, un fard à paupières, un eye-liner, un mascara ou un rouge à lèvre ou qu'elle se présente sous forme d'une crème, d'un lait ou d'un sérum, susceptible d'être utilisé comme produit de soin ou comme produit solaire.

**42.** Procédé de traitement non thérapeutique de la peau ou du cuir chevelu, caractérisé par le fait qu'on applique sur la peau et/ou sur le cuir chevelu une émulsion cosmétique selon l'une quelconque des revendications 28 à 39, et/ou une composition cosmétique selon l'une des revendications 40 et 41.

**43.** Procédé de maquillage de la peau et/ou du cuir chevelu, caractérisé par le fait qu'on applique sur la peau et/ou sur le cuir chevelu une émulsion cosmétique selon l'une quelconque des revendications 28 à 37, et/ou une composition cosmétique selon l'une des revendications 40 et 41.

## Claims

**1.** Cosmetic use of the combination of a silicone surfactant comprising at least one anionic group and of a fatty phase comprising a first oil chosen from volatile oils, from poly($C_1$-$C_{20}$)alkylsiloxanes and from their mixtures in an oil-in-water emulsion in order to decrease the transfer and/or the migration and/or in order to improve the persistence and/or the behaviour of the said emulsion, or of a composition comprising it, on the skin.

**2.** Use of the combination of a silicone surfactant comprising at least one anionic group and of a fatty phase comprising a first oil chosen from volatile oils, from poly($C_1$-$C_{20}$)alkylsiloxanes and from their mixtures for the preparation of an oil-in-water emulsion in order to decrease the transfer and/or the migration and/or in order to improve the persistence and/or the behaviour of the said emulsion, or of a composition comprising it, on the skin.

3. Cosmetic use of the combination of a silicone surfactant comprising at least one anionic group and of a first oil chosen from volatile oils, from poly($C_1$-$C_{20}$)alkylsiloxanes and from their mixtures in an oil-in-water emulsion which does not transfer and/or which does not migrate on the skin.

4. Use according to either of the preceding claims, characterized in that the silicone surfactant containing an anionic group is chosen from surfactants containing a phosphate, sulphate, sulphonate and/or carboxylate group.

5. Use according to any one of the preceding claims, characterized in that the silicone surfactant containing a phosphate group is chosen from those of formula (I):

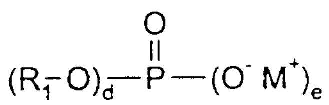

(I)

in which $R_1$ denotes the radical of following formula (V):

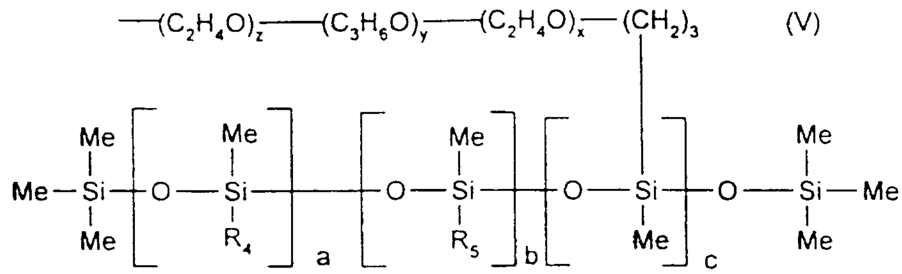

(V)

in which formulae:

Me denotes the methyl radical,
$C_2H_4O$ represents the $-CH_2-CH_2-O-$ group,
$C_3H_6O$ represents the $-CH_2-CH(CH_3)-O-$ group,
a is an integer ranging from 0 to 200,
b is an integer ranging from 0 to 200,
c is an integer ranging from 1 to 200,
$R_4$ denotes a $-(CH_2)_nCH_3$ or phenyl radical, n being an integer ranging from 0 to 10,
$R_5$ denotes the $-(CH_2)_3-(OCH_2CH_2)_x-(OCH_2CH(CH_3))_y-(OCH_2CH_2)_z-OH$ radical,
x, y and z are integers independently ranging from 0 to 20,
d and e range from 1 to 2, with $d + e = 3$,
M is chosen from the group consisting of H, Na, K, Li, $NH_4$ and $N(CH_2CH_2OH)_3$.

6. Use according to any one of Claims 1 to 4, characterized in that the silicone surfactant containing a phosphate group is chosen from those of formula (II):

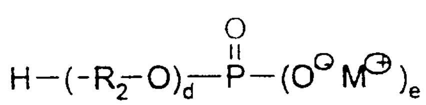

(II)

in which $R_2$ denotes the radical of following formula (VI):

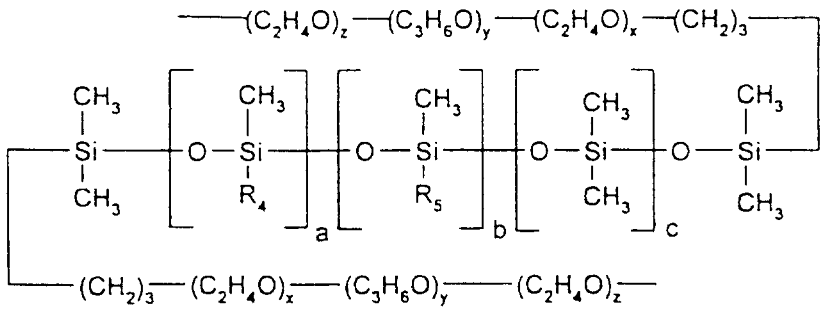

(VI)

in which formulae:

Me denotes the methyl radical,
$C_2H_4O$ represents the $-CH_2-CH_2-O-$ group,
$C_3H_6O$ represents the $-CH_2-CH(CH_3)-O-$ group,
a is an integer ranging from 0 to 200,
b is an integer ranging from 0 to 200,
c is an integer ranging from 1 to 200,
$R_4$ denotes a $-(CH_2)_nCH_3$ or phenyl radical, n being an integer ranging from 0 to 10,
$R_5$ denotes the $-(CH_2)_3-(OCH_2CH_2)_x-(OCH_2CH(CH_3))_y-(OCH_2CH_2)_x-OH$ radical,
x, y and z are integers independently ranging from 0 to 20,
d and e range from 1 to 2, with the proviso that d + e = 3 ,
M is chosen from the group consisting of H, Na, K, Li, $NH_4$ and $N(CH_2CH_2OH)_3$.

7. Use according to any one of Claims 1 to 4, characterized in that the silicone surfactant containing a phosphate group is chosen from those of formula (III):

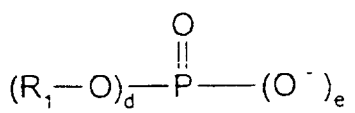 e $[R_3H]$ (III)

in which $R_1$ is the radical of following formula (V):

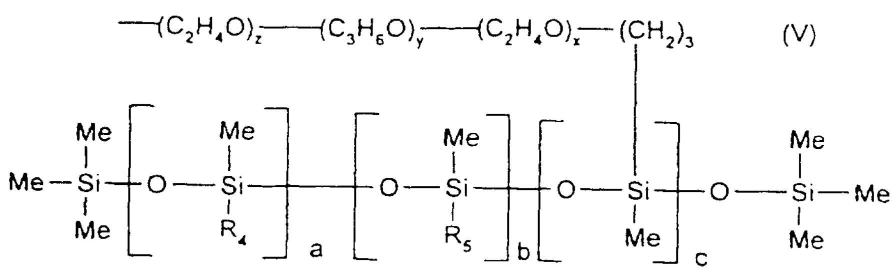

(V)

in which formulae:

Me denotes the methyl radical,
$C_2H_4O$ represents the $-CH_2-CH_2-O-$ group,
$C_3H_6O$ represents the $-CH_2-CH(CH_3)-O-$ group,
a is an integer ranging from 0 to 200,

b is an integer ranging from 0 to 200,
c is an integer ranging from 1 to 200,
$R_4$ denotes a $-(CH_2)_nCH_3$ or phenyl radical, n being an integer ranging from 0 to 10,
$R_5$ denotes the $-(CH_2)_3-(OCH_2CH_2)_x-(OCH_2CH(CH_3))_y-(OCH_2CH_2)_x-OH$ radical,
x, y and z are integers independently ranging from 0 to 20,
d and e range from 1 to 2, with the proviso that d + e = 3 ,
$R_3$ is chosen from:

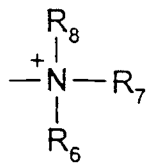

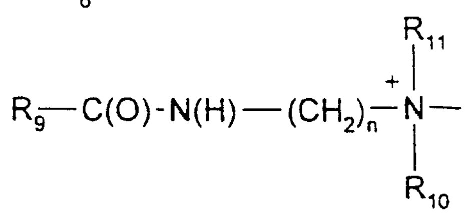

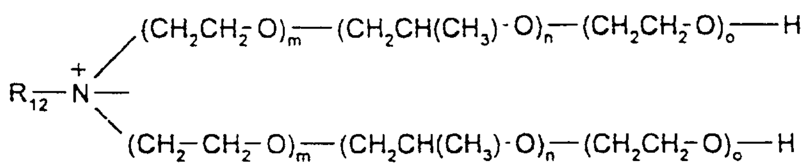

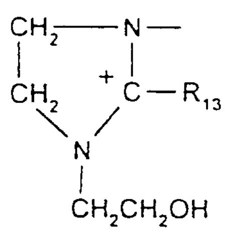

in which formulae:
$R_6$ to $R_9$ independently denote an alkyl radical having from 1 to 20 carbon atoms,
$R_{10}$ and $R_{11}$ independently denote an alkyl radical having from 1 to 3 carbon atoms,
$R_{12}$ and $R_{13}$ independently denote an alkyl radical having from 6 to 20 carbon atoms,
m, n and o independently denote an integer ranging from 0 to 20.

**8.** Use according to any one of Claims 1 to 4, characterized in that the silicone surfactant containing a phosphate group is chosen from those of formula (IV):

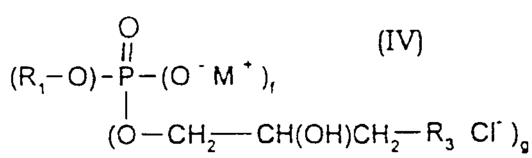

(IV)

in which $R_1$ is the radical of following formula (V):

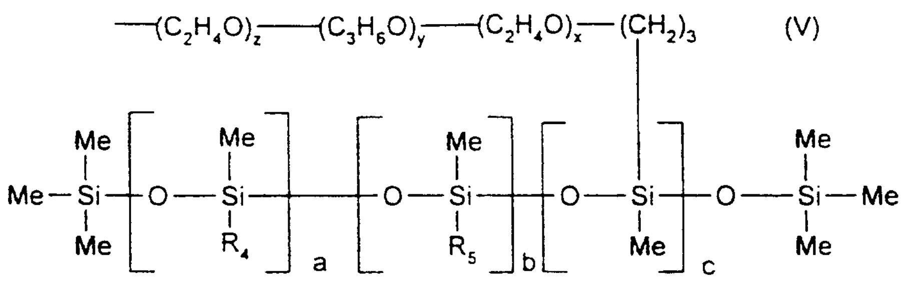

(V)

in which formulae:

Me denotes the methyl radical,
$C_2H_4O$ represents the $-CH_2-CH_2-O-$ group,
$C_3H_6O$ represents the $-CH_2-CH(CH_3)-O-$ group,
a is an integer ranging from 0 to 200,
b is an integer ranging from 0 to 200,
c is an integer ranging from 1 to 200,
$R_4$ denotes a $-(CH_2)_nCH_3$ or phenyl radical, n being an integer ranging from 0 to 10,
$R_5$ denotes the $-(CH_2)_3-(OCH_2CH_2)_x-(OCH_2CH(CH_3))_y-(OCH_2CH_2)_z-OH$ radical,
x, y and z are integers independently ranging from 0 to 20,
f is equal to 0 or 1, g is equal to 1 or 2 and f + g = 2 ,
$R_3$ is chosen from:

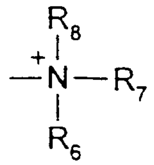

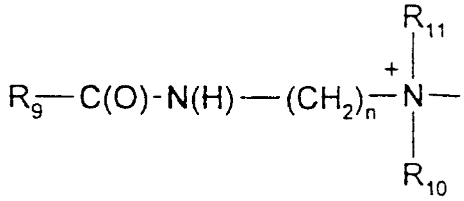

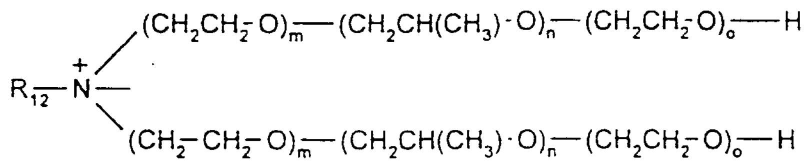

and

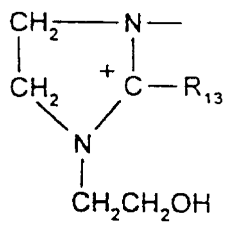

in which formulae:

$R_6$ to $R_9$ independently denote an alkyl radical having from 1 to 20 carbon atoms,
$R_{10}$ and $R_{11}$ independently denote an alkyl radical having from 1 to 3 carbon atoms,
$R_{12}$ and $R_{13}$ independently denote an alkyl radical having from 6 to 20 carbon atoms,
m, n and o independently denote an integer ranging from 0 to 20.

**9.** Use according to any one of Claims 1 to 4, characterized in that the silicone surfactant containing a sulphate group is chosen from those of formula (VII):

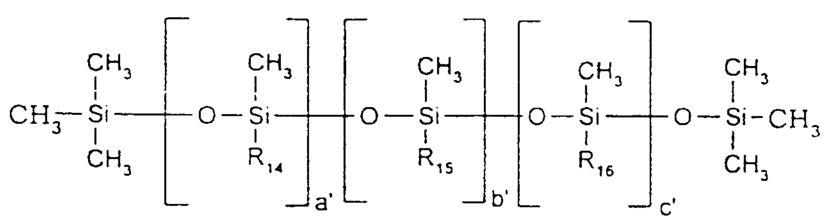 (VII)

in which

$R_{14}$ denotes an alkyl radical having from 1 to 8 carbon atoms or a phenyl radical,
$R_{15}$ denotes a
$-(CH_2)_3-O-(CH_2CH_2O)_u-(CH_2CH(CH_3)-O)_v-(CH_2CH_2O)_w-SO_3^-M^+$ radical,
M is chosen from Na, K, Li and $NH_4$,

$R_{16}$ denotes a $-(CH_2)_3-O-(CH_2CH_2O)_u-(CH_2CH(CH_3)-O)_v-(CH_2CH_2O)_w-H$ radical, in which radical u, v and w independently denote an integer ranging from 0 to 100,
a' and c' independently denote an integer ranging from 0 to 50,
b' denotes an integer ranging from 1 to 50.

**10.** Use according to any one of Claims 1 to 4, characterized in that the silicone surfactant containing a sulphonate group is chosen from those obtained by the reaction of a silicone of formula (VIII):

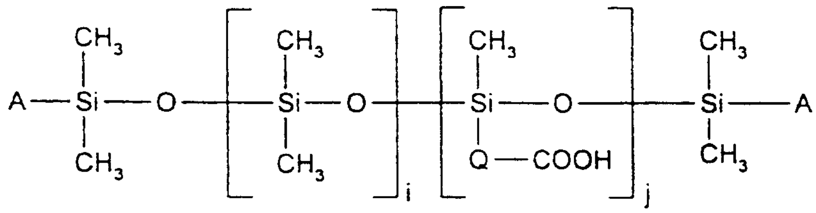
(VIII)

in which

Q denotes $(CH_2)_r$, r being an integer ranging from 3 to 17, j denotes either an integer ranging from 1 to 10 and A denotes a methyl radical or j = 0 and A denotes a -Q-COOH radical,
i denotes an integer ranging from 1 to 200,
with a taurine derivative of formula $R_{17}-NH-(CH_2)_2-SO_3M$, in which $R_{17}$ denotes an alkyl radical having from 1 to 40 carbon atoms, and
M is chosen from Na, K, Li and $NH_4$.

**11.** Use according to any one of Claims 1 to 4, characterized in that the silicone surfactant containing a sulphonate group is chosen from those of formula (IX):

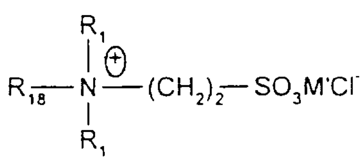
(IX)

in which

$R_{18}$ denotes an alkyl radical having from 1 to 40 carbon atoms,
$R_1$ denotes the radical of following formula (V):

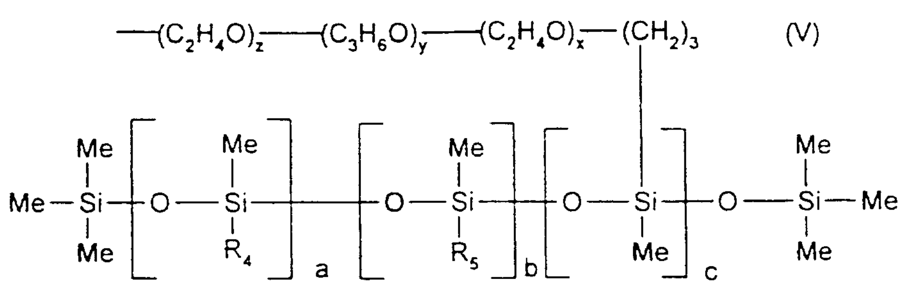
(V)

in which formulae:
Me denotes the methyl radical,
$C_2H_4O$ represents the $-CH_2-CH_2-O-$ group,
$C_3H_6O$ represents the $-CH_2-CH(CH_3)-O-$ group,

a is an integer ranging from 0 to 200,
b is an integer ranging from 0 to 200,
c is an integer ranging from 1 to 200,
$R_4$ denotes a $-(CH_2)_nCH_3$ or phenyl radical, n being an integer ranging from 0 to 10,
$R_5$ denotes the $-(CH_2)_3-(OCH_2CH_2)_x-(OCH_2CH(CH_3))_y-(OCH_2CH_2)_z-OH$ radical,
x, y and z are integers independently ranging from 0 to 20,
M' is chosen from Na, K, Li and $NH_4$.

**12.** Use according to any one of the preceding claims, characterized in that the said first oil is present in the proportion of at least 65% by weight with respect to the total weight of the fatty phase of the emulsion.

**13.** Use according to any one of the preceding claims, characterized in that the volatile oil is chosen from volatile hydrocarbon oils, volatile silicone oils and their mixtures.

**14.** Use according to any one of the preceding claims, characterized in that the volatile oil is chosen from isoparaffins, volatile cyclic silicones having from 3 to 8 silicon atoms and preferably from 4 to 6, cyclocopolymers of the dimethylsiloxane/methylalkylsiloxane type, volatile linear silicones having from 2 to 9 silicon atoms and their mixtures.

**15.** Use according to any one of the preceding claims, characterized in that the volatile oil is chosen from isododecane, cyclotetradimethylsiloxane, cyclopentadimethylsiloxane, cyclohexadimethylsiloxane, the dimethylsiloxane/methyloctylsiloxane cyclocopolymer, hexamethyldisiloxane, hexylheptamethyltrisiloxane, octylheptamethyltrisiloxane and their mixtures.

**16.** Use according to any one of the preceding claims, characterized in that the poly($C_1$-$C_{20}$)alkylsiloxanes have a viscosity at 25°C of less than or equal to 0.06 $m^2/s$.

**17.** Use according to any one of the preceding claims, characterized in that the poly($C_1$-$C_{20}$)alkylsiloxanes are chosen from alkylmethylpolysiloxanes, linear polydimethylsiloxanes and their mixtures.

**18.** Use according to one of Claims 1 to 17, characterized in that the fatty phase additionally comprises at least one other fatty substance, other than the said first oil, at a content of less than or equal to 7% by weight with respect to the total weight of the emulsion.

**19.** Use according to Claim 18, characterized in that the fatty phase comprises from 65% to 98% by weight, with respect to the total weight of the fatty phase, of the said first oil and from 2% to 35% by weight of the said other fatty substance.

**20.** Use according to either of Claims 18 and 19, characterized in that the said other fatty substance is chosen from vegetable, mineral, animal and/or synthetic oils, pasty fatty substances, gums and/or waxes.

**21.** Use according to any one of the preceding claims, characterized in that the fatty phase comprises at least 75% by weight, with respect to the total weight of the fatty phase, of oil chosen from volatile oils, from poly($C_1$-$C_{20}$)alkylsiloxanes and from their mixtures.

**22.** Use according to any one of Claims 1 to 17, characterized in that the fatty phase comprises 100% by weight, with respect to the total weight of the fatty phase, of the said first oil.

**23.** Use according to any one of the preceding claims, characterized in that the emulsion comprises, with respect to the total weight of the emulsion, from 5% to 40% by weight of fatty phase, from 0.5% to 15% by weight of silicone surfactant containing an anionic group and from 15% to 94.5% by weight of aqueous phase.

**24.** Use according to any one of the preceding claims, characterized in that the emulsion comprises, with respect to the total weight of the emulsion, from 12% to 30% by weight of fatty phase, from 3% to 6% by weight of silicone surfactant containing an anionic group and from 40% to 70% by weight of aqueous phase.

**25.** Use according to any one of the preceding claims, characterized in that the emulsion is comprised within a cosmetic composition.

**26.** Use according to Claim 2, characterized in that the emulsion is comprised within a cosmetic, pharmaceutical or hygienic composition.

**27.** Use according to Claim 25 or 26, characterized in that the composition is provided in the form of a make-up product, such as a foundation, a blusher, an eyeshadow, an eyeliner, a mascara or a lipstick, or is provided in the form of a cream, of a milk or of a serum which is capable of being used as care product or anti-sun product.

**28.** Oil-in-water emulsion comprising:

- (a) an aqueous phase,
- (b) a fatty phase comprising at least one first oil chosen from volatile oils, from poly($C_1$-$C_{20}$)alkylsiloxanes and from their mixtures at a content of at least 65% by weight with respect to the total weight of the fatty phase,
- (c) a silicone surfactant comprising at least one anionic group chosen from:

  - (i) silicone surfactants containing a phosphate group of formula (I), (II), (III) or (IV):

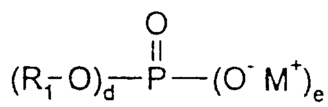

(I)

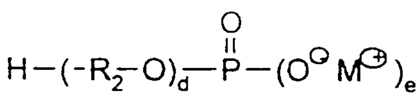

(II)

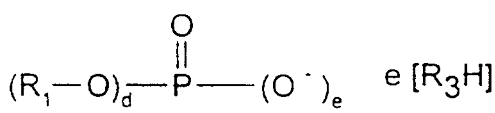

(III)

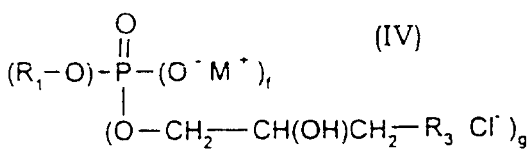

(IV)

in which formulae:

$R_1$ denotes the radical of following formula (V):

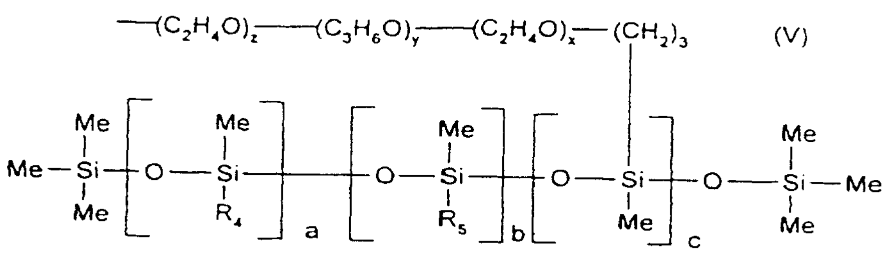

(V)

$R_2$ denotes the radical of following formula (VI):

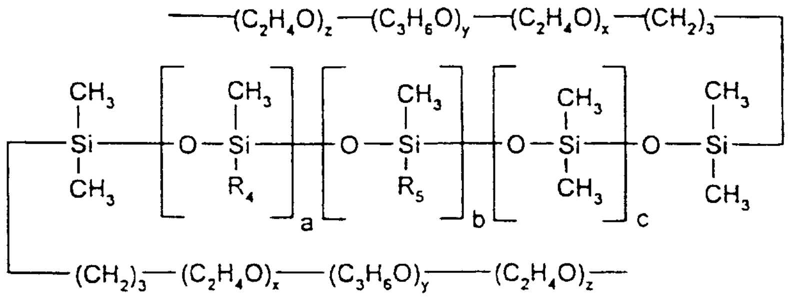

(VI)

in which formula:
Me denotes the methyl radical,
$C_2H_4O$ represents the $-CH_2-CH_2-O-$ group,
$C_3H_6O$ represents the $-CH_2-CH(CH_3)-O-$ group,
a is an integer ranging from 0 to 200,
b is an integer ranging from 0 to 200,
c is an integer ranging from 1 to 200,
$R_4$ denotes a $-(CH_2)_nCH_3$ or phenyl radical, n being an integer ranging from 0 to 10,
$R_5$ denotes the $-(CH_2)_3-(OCH_2CH_2)_x-(OCH_2CH(CH_3))_y-(OCH_2CH_2)_z-OH$ radical,
x, y and z are integers independently ranging from 0 to 20,
$R_3$ is chosen from:

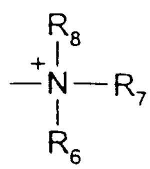

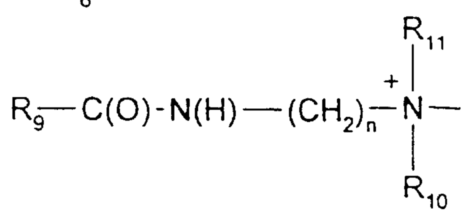

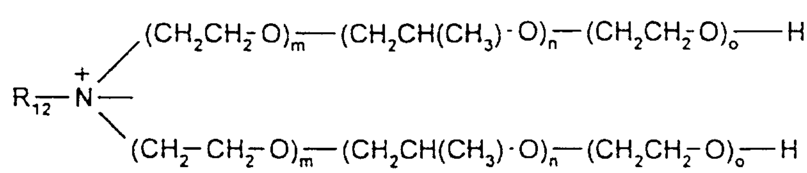

and

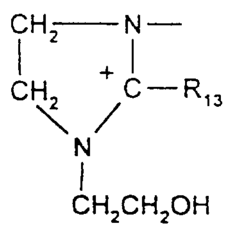

in which formulae:

$R_6$ to $R_9$ independently denote an alkyl radical having from 1 to 20 carbon atoms,
$R_{10}$ and $R_{11}$ independently denote an alkyl radical having from 1 to 3 carbon atoms,
$R_{12}$ and $R_{13}$ independently denote an alkyl radical having from 6 to 20 carbon atoms,
m, n and o independently denote an integer ranging from 0 to 20,
d and e range from 1 to 2, with $d + e = 3$ ,
f is equal to 0 or 1, g is equal to 1 or 2 and $f + g = 2$ ,
M is chosen from the group consisting of H, Na, K, Li, $NH_4$ and $N(CH_2CH_2OH)_3$,
(ii) silicone surfactants containing a sulphate group of formula (VII):

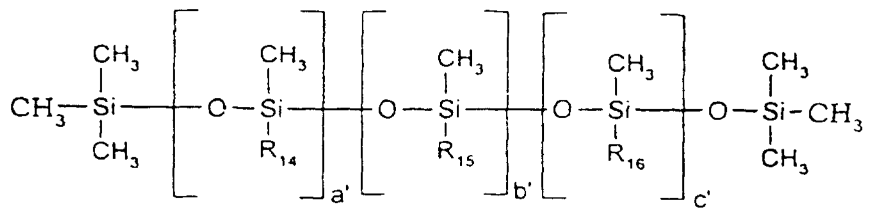

(VII)

in which
$R_{14}$ denotes an alkyl radical having from 1 to 8 carbon atoms or a phenyl radical,
$R_{15}$ denotes a
$-(CH_2)_3-O-(CH_2CH_2O)_u-(CH_2CH(CH_3)-O)_v-(CH_2CH_2O)_w-SO_3^-M'^+$ radical,
M' is chosen from Na, K, Li and $NH_4$,
$R_{16}$ denotes a
$-(CH_2)_3-O-(CH_2CH_2O)_u-(CH_2CH(CH_3)-O)_v-(CH_2CH_2O)_w-H$ radical,
in which radical u, v and w independently denote an integer ranging from 0 to 100,
a' and c' independently denote an integer ranging from 0 to 50,
b' denotes an integer ranging from 1 to 50,

- (iii) silicone surfactants containing a sulphonate group obtained by the reaction of a silicone of formula (VIII):

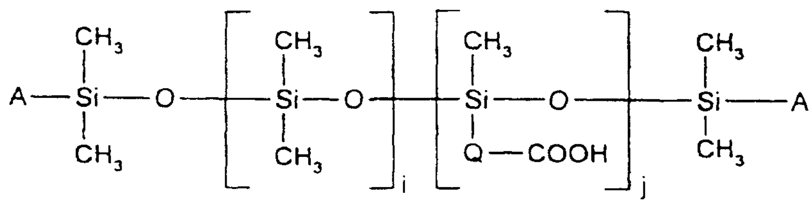

(VIII)

in which

Q denotes $(CH_2)_r$, r being an integer ranging from 3 to 17,
j denotes either an integer ranging from 1 to 10 and A denotes a methyl radical or j = 0 and A denotes a -Q-COOH radical,
i denotes an integer ranging from 1 to 200,
with a taurine derivative of formula $R_{17}-NH-(CH_2)_2-SO_3M''$,
in which $R_{17}$ denotes an alkyl radical having from 1 to 40 carbon atoms, and
M'' is chosen from Na, K, Li and $NH_4$,

- (iv) silicone surfactants containing a sulphonate group of formula (IX):

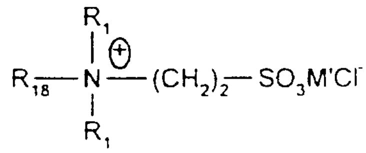

(IX)

in which

$R_{18}$ denotes an alkyl radical having from 1 to 40 carbon atoms,
$R_1$ denotes the radical of formula (V) as defined for the compound of formula (I),
M' is chosen from Na, K, Li and $NH_4$,
with the proviso that, when the silicone surfactant is of formula (I), the fatty phase of the emulsion comprises another fatty substance, other than volatile oils and other than poly($C_1$-$C_{20}$)alkylsiloxanes, at a content of less than or equal to 7% by weight with respect to the total weight of the emulsion.

**29.** Emulsion according to Claim 28, characterized in that the volatile oil is chosen from volatile hydrocarbon oils, volatile silicone oils and their mixtures.

**30.** Emulsion according to either of Claims 28 and 29, characterized in that the volatile oil is chosen from isoparaffins, volatile cyclic silicones having from 3 to 8 silicon atoms and preferably from 4 to 6, cyclocopolymers of the dimethylsiloxane/methylalkylsiloxane type, volatile linear silicones having from 2 to 9 silicon atoms and their mixtures.

**31.** Emulsion according to one of Claims 28 to 30, characterized in that the volatile oil is chosen from isododecane, cyclotetradimethylsiloxane, cyclopentadimethylsiloxane, cyclohexadimethylsiloxane, the dimethylsiloxane/methyloctylsiloxane cyclocopolymer, hexamethyldisiloxane, hexylheptamethyltrisiloxane, octylheptamethyltrisiloxane and their mixtures.

**32.** Emulsion according to any one of Claims 28 to 31, characterized in that the poly($C_1$-$C_{20}$)alkyl-siloxanes have a viscosity at 25°C of less than or equal to 0.06 $m^2$/s.

**33.** Emulsion according to any one of Claims 28 to 32, characterized in that the poly($C_1$-$C_{20}$)alkyl-siloxanes are chosen from alkylmethylpolysiloxanes, linear polydimethylsiloxanes and their mixtures.

**34.** Emulsion according to any one of Claims 28 to 33, characterized in that the fatty phase comprises at least 75% by weight, with respect to the total weight of the fatty phase, of oil chosen from volatile oils, from poly($C_1$-$C_{20}$)alkylsiloxanes and from their mixtures.

**35.** Emulsion according to any one of Claims 28 to 34, characterized in that the fatty phase comprises 100% by weight, with respect to the total weight of the fatty phase, of the said first oil, when the said silicone surfactant is other than those of formula (I).

**36.** Emulsion according to one of Claims 28 to 35, characterized in that the fatty phase additionally comprises at least one other fatty substance, other than the said first oil, at a content of less than or equal to 7% by weight with respect to the total weight of the emulsion.

**37.** Emulsion according to one of Claims 28 to 36, characterized in that the said other fatty substance is chosen from vegetable, mineral, animal and/or synthetic oils, pasty fatty substances, gums and/or waxes.

**38.** Emulsion according to one of Claims 28 to 37, characterized in that it comprises, with respect to the total weight of the emulsion, from 5% to 40% by weight of fatty phase, from 0.5% to 15% by weight of silicone surfactant containing an anionic group and from 15% to 94.5% by weight of aqueous phase.

**39.** Emulsion according to one of Claims 28 to 37, characterized in that it comprises, with respect to the total weight of the emulsion, from 12% to 30% by weight of fatty phase, from 3% to 6% by weight of silicone surfactant containing an anionic group and from 40% to 70% by weight of aqueous phase.

40. Cosmetic, pharmaceutical or hygienic composition, characterized in that it comprises an emulsion according to one of Claims 28 to 39.

41. Composition according to Claim 40, characterized in that it is provided in the form of a make-up product, such as a foundation, a blusher, an eyeshadow, an eyeliner, a mascara or a lipstick, or in that it is provided in the form of a cream, of a milk or of a serum which is capable of being used as care product or anti-sun product.

42. Process for the non-therapeutic treatment of the skin or of the scalp, characterized in that a cosmetic emulsion according to any one of Claims 28 to 39 and/or a cosmetic composition according to either of Claims 40 and 41 is applied on the skin and/or on the scalp.

43. Process for making up the skin and/or the scalp, characterized in that a cosmetic emulsion according to any one of Claims 28 to 39 and/or a cosmetic composition according to either of Claims 40 and 41 is applied on the skin and/or on the scalp.

**Patentansprüche**

1. Kosmetische Verwendung der Kombination eines grenzflächenaktiven Silicons, das mindestens eine anionische Gruppe aufweist, und einer Fettphase, die ein erstes Öl, das unter den flüchtigen Ölen, den $C_{1-20}$-Polyalkylsiloxanen und deren Gemischen ausgewählt ist, enthält, in einer Öl-in-Wasser-Emulsion, um den Transfer und/oder die Migration zu vermindern und/oder die Haftung und/oder die Haltbarkeit der Emulsion oder einer sie enthaltenden Zusammensetzung auf der Haut zu verbessern.

2. Verwendung der Kombination eines grenzflächenaktiven Silicons, das mindestens eine anionische Gruppe aufweist, und einer Fettphase, die ein erstes Öl, das unter den flüchtigen Ölen, den $C_{1-20}$-Polyalkylsiloxanen und deren Gemischen ausgewählt ist, enthält, zur Herstellung einer Öl-in-Wasser-Emulsion, um den Transfer und/oder die Migration zu vermindern und/oder die Haftung und/oder die Haltbarkeit der Emulsion oder einer sie enthaltenden Zusammensetzung auf der Haut zu verbessern.

3. Kosmetische Verwendung der Kombination eines grenzflächenaktiven Silicons, das mindestens eine anionische Gruppe aufweist, und eines ersten Öls, das unter den flüchtigen Ölen, den $C_{1-20}$-Polyalkylsiloxanen und deren Gemischen ausgewählt ist, in einer Öl-in-Wasser-Emulsion, die sich nicht überträgt und/oder keine Migration auf der Haut zeigt.

4. Verwendung nach einem der vorhergehenden Ansprüche, dadurch gekennzeichnet, daß das grenzflächenaktive Silicon mit anionischer Gruppe unter den grenzflächenaktiven Stoffen mit Phosphatgruppe, Sulfatgruppe, Sulfonatgruppe und/oder Carboxylatgruppe ausgewählt ist.

5. Verwendung nach einem der vorhergehenden Ansprüche, dadurch gekennzeichnet, daß das grenzflächenaktive Silicon mit Phosphatgruppe unter den Verbindungen der Formel I ausgewählt ist:

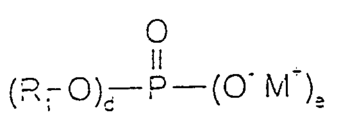

(I)

wobei $R_1$ die Gruppe der folgenden Formel V bedeutet:

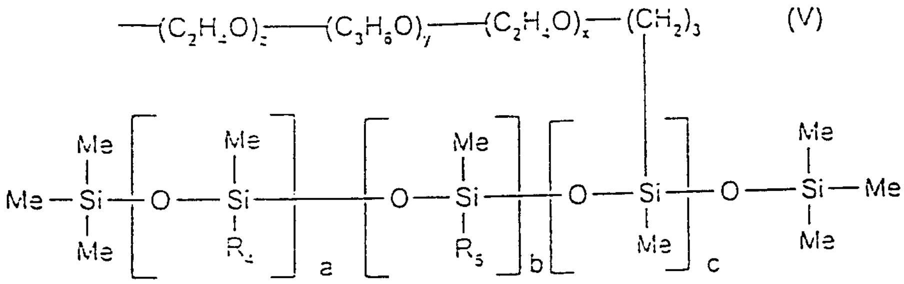

(V)

worin bedeuten:

- Me die Methylgruppe,
- $C_2H_4O$ die Gruppe $-CH_2-CH_2-O-$,
- $C_3H_6O$ die Gruppe $-CH_2-CH(CH_3)-O-$,
- a 0 oder eine ganze Zahl im Bereich von 1 bis 200,
- b 0 oder eine ganze Zahl im Bereich von 1 bis 200,
- c eine ganze Zahl im Bereich von 1 bis 200,
- $R_4$ eine Gruppe $-(CH_2)_nCH_3$ oder Phenyl,
  wobei n 0 oder eine ganze Zahl im Bereich von 1 bis 10 bedeutet,
- $R_5$ die Gruppe
  $-(CH_2)_3-(OCH_2CH_2)_x-(OCH_2CH(CH_3))_y-(OCH_2CH_2)_z-OH$, wobei x, y und z unabhängig voneinander 0 oder ganze Zahlen im Bereich von 1 bis 20 bedeuten,
  und

  - d und e 1 oder 2 sind, mit d+e=3 ,
  - M H, Na, K, Li, $NH_4$ oder $N(CH_2CH_2OH)_3$.

**6.** Verwendung nach einem der Ansprüche 1 bis 4, dadurch gekennzeichnet, daß das grenzflächenaktive Silicon mit Phosphatgruppe unter den Verbindungen der Formel II ausgewählt ist:

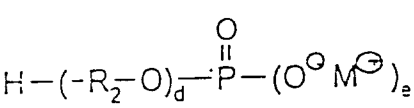

(II)

wobei $R_2$ die Gruppe der folgenden Formel VI bedeutet:

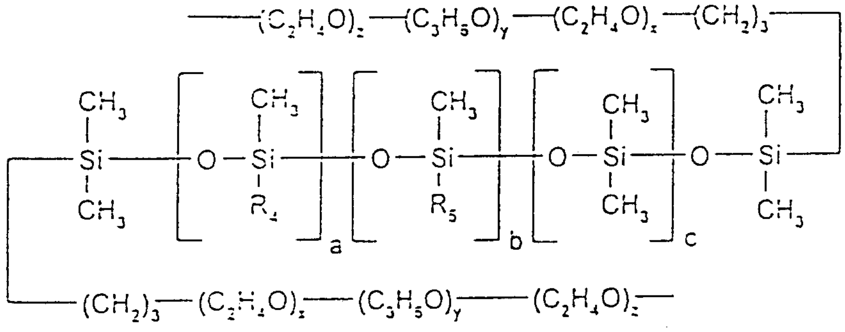

(VI),

worin bedeuten:

- Me die Methylgruppe,
- $C_2H_4O$ die Gruppe $-CH_2-CH_2-O-$,
- $C_3H_6O$ die Gruppe $-CH_2-CH(CH_3)-O-$,
- a 0 oder eine ganze Zahl im Bereich von 1 bis 200,
- b 0 oder eine ganze Zahl im Bereich von 1 bis 200,
- c eine ganze Zahl im Bereich von 1 bis 200,
- $R_4$ eine Gruppe $-(CH_2)_nCH_3$ oder Phenyl, wobei n 0 oder eine ganze Zahl im Bereich von 1 bis 10 bedeutet,
- $R_5$ die Gruppe $-(CH_2)_3-(OCH_2CH_2)_x-(OCH_2CH(CH_3))_y-(OCH_2CH_2)_z-OH$, wobei x, y und z unabhängig voneinander 0 oder ganze Zahlen im Bereich von 1 bis 20 bedeuten,

  - d und e 1 oder 2, mit der Maßgabe, daß d+e=3 ,
  - M H, Na, K, Li, $NH_4$ oder $N(CH_2CH_2OH)_3$.

**7.** Verwendung nach einem der Ansprüche 1 bis 4, dadurch gekennzeichnet, daß das grenzflächenaktive Silicon mit Phosphatgruppe unter den Verbindungen der Formel III ausgewählt ist:

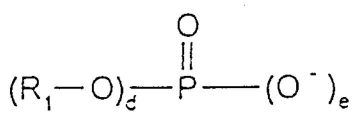

e $[R_3H]$ (III)

wobei $R_1$ die Gruppe der folgenden Formel V bedeutet:

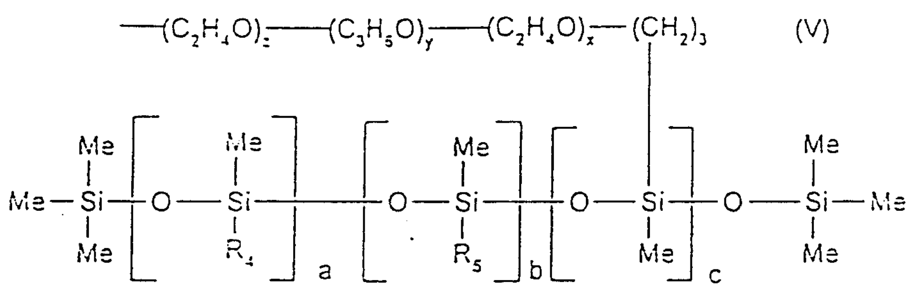

(V)

,

worin bedeuten:

- Me die Methylgruppe,

- $C_2H_4O$ die Gruppe $-CH_2-CH_2-O-$,
- $C_3H_6O$ die Gruppe $-CH_2-CH(CH_3)-O-$,
- a 0 oder eine ganze Zahl im Bereich von 1 bis 200,
- b 0 oder eine ganze Zahl im Bereich von 1 bis 200,
- c eine ganze Zahl im Bereich von 1 bis 200,
- $R_4$ eine Gruppe $-(CH_2)_nCH_3$ oder Phenyl, wobei n 0 oder eine ganze Zahl im Bereich von 1 bis 10 bedeutet,
- $R_5$ die Gruppe $-(CH_2)_3-(OCH_2CH_2)_x-(OCH_2CH(CH_3))_y-(OCH_2CH_2)_z-OH$, wobei x, y und z unabhängig voneinander 0 oder ganze Zahlen im Bereich von 1 bis 20 bedeuten,

- d und e 1 oder 2, mit der Maßgabe, daß d+e=3 ,
- $R_3$:

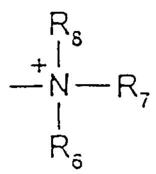

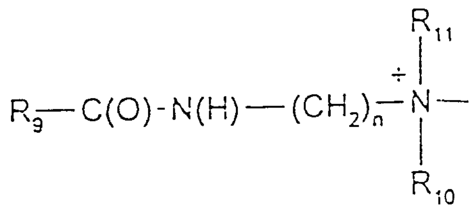

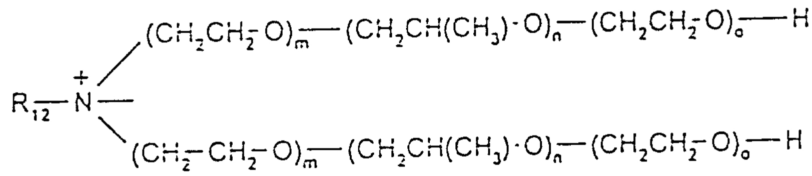

und

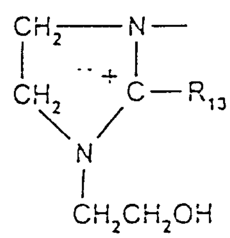

,

worin bedeuten:

- $R_6$ bis $R_9$ unabhängig voneinander eine Alkylgruppe mit 1 bis 20 Kohlenstoffatomen,
- $R_{10}$ und $R_{11}$ unabhängig voneinander eine Alkylgruppe mit 1 bis 3 Kohlenstoffatomen,
- $R_{12}$ und $R_{13}$ unabhängig voneinander eine Alkylgruppe mit 6 bis 20 Kohlenstoffatomen,
- m, n und o unabhängig voneinander 0 oder eine ganze Zahl im Bereich von 1 bis 20.

**8.** Verwendung nach einem der Ansprüche 1 bis 4, dadurch gekennzeichnet, daß das grenzflächenaktive Silicon mit Phosphatgruppe unter den Verbindungen der Formel IV ausgewählt ist:

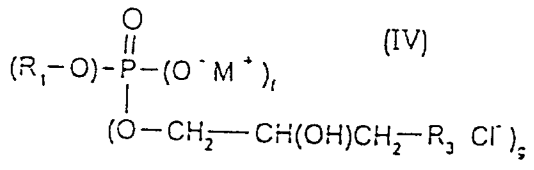

(IV)

wobei $R_1$ die Gruppe der folgenden Formel V bedeutet:

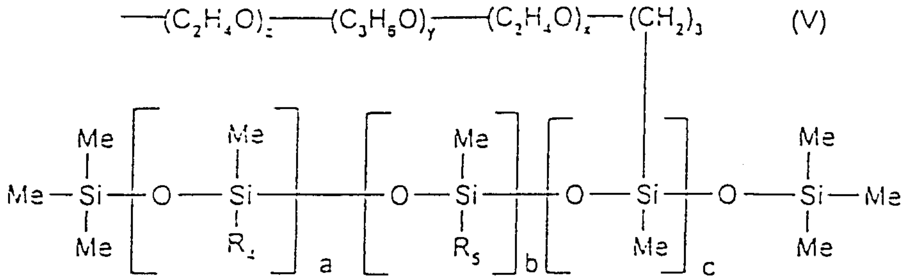

(V)

worin bedeuten:

- Me die Methylgruppe,
- $C_2H_4O$ die Gruppe $-CH_2-CH_2-O-$,
- $C_3H_6O$ die Gruppe $-CH_2-CH(CH_3)-O-$,
- a 0 oder eine ganze Zahl im Bereich von 1 bis 200,
- b 0 oder eine ganze Zahl im Bereich von 1 bis 200,
- c eine ganze Zahl im Bereich von 1 bis 200,
- $R_4$ eine Gruppe $-(CH_2)_nCH_3$ oder Phenyl, wobei n 0 oder eine ganze Zahl im Bereich von 1 bis 10 bedeutet,
- $R_5$ die Gruppe $-(CH_2)_3-(OCH_2CH_2)_x-(OCH_2CH(CH_3))_y-(OCH_2CH_2)_z-OH$, wobei x, y und z unabhängig voneinander 0 oder ganze Zahlen im Bereich von 1 bis 20 bedeuten,

- f 0 oder 1, g 1 oder 2 und f+g=2 ,
- $R_3$:

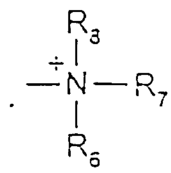

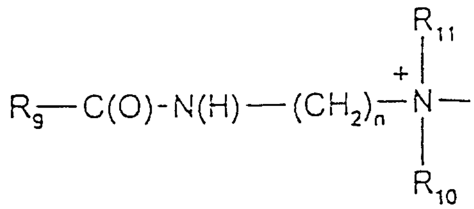

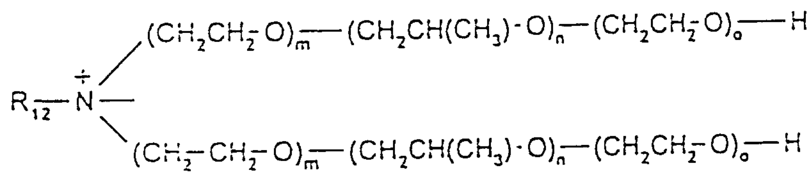

und 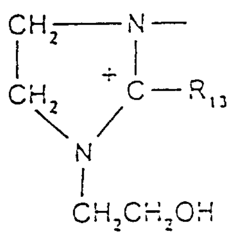 ,

worin bedeuten:

- $R_6$ bis $R_9$ unabhängig voneinander eine Alkylgruppe mit 1 bis 20 Kohlenstoffatomen,
- $R_{10}$ und $R_{11}$ unabhängig voneinander eine Alkylgruppe mit 1 bis 3 Kohlenstoffatomen,
- $R_{12}$ und $R_{13}$ unabhängig voneinander eine Alkylgruppe mit 6 bis 20 Kohlenstoffatomen,
- m, n und o unabhängig voneinander 0 oder eine ganze Zahl im Bereich von 1 bis 20.

**9.** Verwendung nach einem der Ansprüche 1 bis 4, dadurch gekennzeichnet, daß das grenzflächenaktive Silicon mit Sulfatgruppe unter den Verbindungen der Formel VII ausgewählt ist:

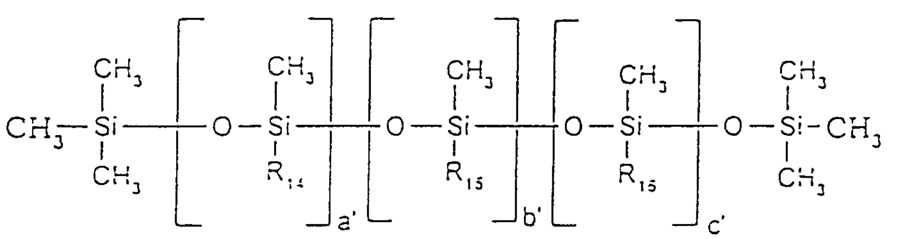 (VII)

worin bedeuten:

- $R_{14}$ eine Alkylgruppe mit 1 bis 8 Kohlenstoffatomen oder eine Phenylgruppe,
- $R_{15}$ eine Gruppe

-$(CH_2)_3$-O-$(CH_2CH_2O)_u$-$(CH_2CH(CH_3)$-$O)_v$-$(CH_2CH_2O)_w$-$SO_3^-M^-$,
wobei M unter Na, K, Li und $NH_4$ ausgewählt ist,

- $R_{16}$ eine Gruppe
  -$(CH_2)_3$-O-$(CH_2CH_2O)_u$-$(CH_2CH(CH_3)$-$O)_v$-$(CH_2CH_2O)_w$-H,
  wobei u, v und w unabhängig voneinander 0 oder eine ganze Zahl im Bereich von 1 bis 100 bedeuten,
- a' und c' unabhängig voneinander 0 oder eine ganze Zahl in Bereich von 1 bis 50,
- b' eine ganze Zahl im Bereich von 1 bis 50.

**10.** Verwendung nach einem der Ansprüche 1 bis 4, dadurch gekennzeichnet, daß das grenzflächenaktive Silicon mit Sulfonatgruppe unter den Verbindungen ausgewählt ist, die durch Umsetzung eines Silicons der Formel VIII

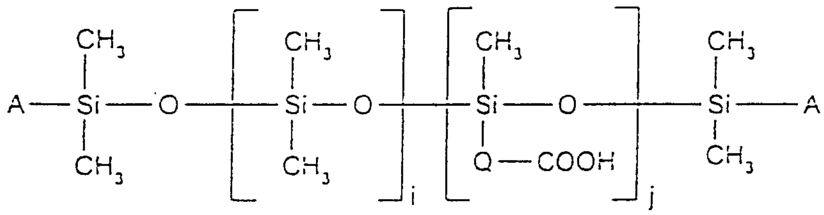

(VIII)

worin bedeuten:

Q $(CH_2)_r$, wobei r eine ganze Zahl im Bereich von 3 bis 17 ist,
j entweder eine ganze Zahl im Bereich von 1 bis 10 und A eine Methylgruppe oder j = 0 und A eine Gruppe -Q-COOH,
i eine ganze Zahl im Bereich von 1 bis 200
mit einem Taurinderivat der Formel $R_{17}$-NH-$(CH_2)_2$-$SO_3M$ hergestellt ist, wobei $R_{17}$ eine Alkylgruppe mit 1 bis 40 Kohlenstoffatomen bedeutet und M unter Na, K, Li und $NH_4$ ausgewählt ist.

**11.** Verwendung nach einem der Ansprüche 1 bis 4, dadurch gekennzeichnet, daß das grenzflächenaktive Silicon mit Sulfonatgruppe unter den Verbindungen der Formel IX ausgewählt ist

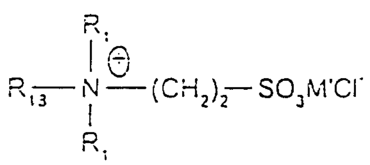

(IX)

worin bedeuten:

$R_{18}$ eine Alkylgruppe mit 1 bis 40 Kohlenstoffatomen,
$R_1$ die Gruppe der folgenden Formel V:

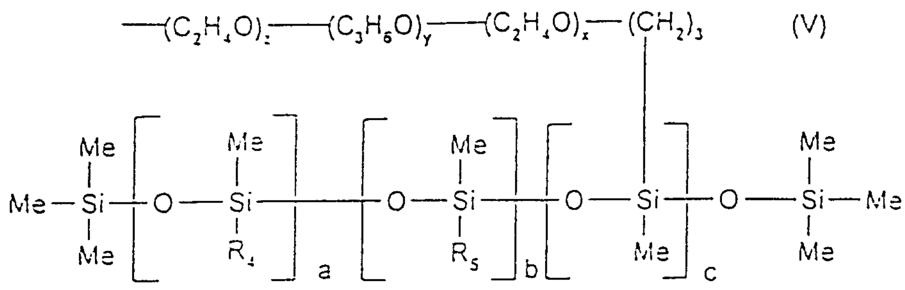

(V)

worin bedeuten:

- Me die Methylgruppe,
- $C_2H_4O$ die Gruppe $-CH_2-CH_2-O-$,
- $C_3H_6O$ die Gruppe $-CH_2-CH(CH_3)-O-$,
- a 0 oder eine ganze Zahl im Bereich von 1 bis 200,
- b 0 oder eine ganze Zahl im Bereich von 1 bis 200,
- c eine ganze Zahl im Bereich von 1 bis 200,
- $R_4$ eine Gruppe $-(CH_2)_nCH_3$ oder Phenyl, wobei n 0 oder eine ganze Zahl im Bereich von 1 bis 10 bedeutet,
- $R_5$ die Gruppe $-(CH_2)_3-(OCH_2CH_2)_x-(OCH_2CH(CH_3))_y-(OCH_2CH_2)_z-OH$, wobei x, y und z unabhängig voneinander 0 oder ganze Zahlen von 1 bis 20 bedeuten,
- M' Na, K, Li oder $NH_4$.

**12.** Verwendung nach einem der vorhergehenden Ansprüche, dadurch gekennzeichnet, daß das erste Öl in einem Mengenanteil von mindestens 65 Gew.-%, bezogen auf das Gesamtgewicht der Fettphase der Emulsion, vorliegt.

**13.** Verwendung nach einem der vorhergehenden Ansprüche, dadurch gekennzeichnet, daß das flüchtige Öl unter den flüchtigen Kohlenwasserstoffölen, den flüchtigen Siliconölen und deren Gemischen ausgewählt ist.

**14.** Verwendung nach einem der vorhergehenden Ansprüche, dadurch gekennzeichnet, daß das flüchtige Öl unter den Isoparaffinen, den cyclischen flüchtigen Siliconen mit 3 bis 8 Siliciumatomen und vorzugsweise 4 bis 6 Siliciumatomen, den Cyclocopolymeren vom Dimethylsiloxan/Methylalkylsiloxan-Typ, den geradkettigen flüchtigen Siliconen mit 2 bis 9 Siliciumatomen und deren Gemischen ausgewählt ist.

**15.** Verwendung nach einem der vorhergehenden Ansprüche, dadurch gekennzeichnet, daß das flüchtige Öl unter Isododecan, Cyclotetradimethylsiloxan, Cyclopentadimethylsiloxan, Cyclohexadimethylsiloxan, Dimethylsiloxan/Methyloctylsiloxan-Cyclocopolymer, Hexamethyldisiloxan, Hexylheptamethyltrisiloxan, Octylheptamethyltrisiloxan und deren Gemischen ausgewählt ist.

**16.** Verwendung nach einem der vorhergehenden Ansprüche, dadurch gekennzeichnet, daß die $C_{1-20}$-Polyalkylsiloxane eine Viskosität bei 25 °C von höchstens 0,06 $m^2/s$ aufweisen.

**17.** Verwendung nach einem der vorhergehenden Ansprüche, dadurch gekennzeichnet, daß die $C_{1-20}$-Polyalkylsiloxane unter den geradkettigen Polydimethylsiloxanen, den Alkylmethylpolysiloxanen und deren Gemischen ausgewählt sind.

**18.** Verwendung nach einem der Ansprüche 1 bis 17, dadurch gekennzeichnet, daß die Fettphase ferner mindestens eine weitere Fettsubstanz, die von dem ersten Öl verschieden ist, in einem Mengenanteil von höchstens 7 Gew.-%, bezogen auf das Gesamtgewicht der Emulsion, enthält.

**19.** Verwendung nach Anspruch 18, dadurch gekennzeichnet, daß die Fettphase 65 bis 98 Gew.-% erstes Öl und 2 bis 35 Gew.-% der weiteren Fettsubstanz, bezogen auf das Gesamtgewicht der Fettphase, enthält.

**20.** Verwendung nach einem der Ansprüche 18 und 19, dadurch gekennzeichnet, daß die weitere Fettsubstanz unter den pflanzlichen, mineralischen, tierischen und/oder synthetischen Ölen, pastösen Fettsubstanzen, Gummen und/oder Wachsen ausgewählt ist.

**21.** Verwendung nach einem der vorhergehenden Ansprüche, dadurch gekennzeichnet, daß die Fettphase mindestens 75 Gew.-% Öl, das unter den flüchtigen Ölen, den $C_{1-20}$-Polyalkylsiloxanen und deren Gemischen ausgewählt ist, bezogen auf das Gesamtgewicht der Fettphase, enthält.

**22.** Verwendung nach einem der Ansprüche 1 bis 17, dadurch gekennzeichnet, daß die Fettphase 100 Gew.-% erstes Öl, bezogen auf das Gesamtgewicht der Fettphase, enthält.

**23.** Verwendung nach einem der vorhergehenden Ansprüche, dadurch gekennzeichnet, daß die Emulsion 5 bis 40 Gew.-% Fettphase, 0,5 bis 15 Gew.-% grenzflächenaktives Silicon mit anionischer Gruppe und 15 bis 94,5 Gew.-% wäßrige Phase, bezogen auf das Gesamtgewicht der Emulsion, enthält.

**24.** Verwendung nach einem der vorhergehenden Ansprüche, dadurch gekennzeichnet, daß die Emulsion 12 bis 30 Gew.-% Fettphase, 3 bis 6 Gew.-% grenzflächenaktives Silicon mit anionischer Gruppe und 40 bis 70 Gew.-% wäßrige Phase, bezogen auf das Gesamtgewicht der Emulsion, enthält.

**25.** Verwendung nach einem der vorhergehenden Ansprüche, dadurch gekennzeichnet, daß die Emulsion in einer kosmetischen Zusammensetzung enthalten ist.

**26.** Verwendung nach Anspruch 2, dadurch gekennzeichnet, daß die Emulsion in einer kosmetischen, pharmazeutischen oder hygienischen Zusammensetzung enthalten ist.

**27.** Verwendung nach Anspruch 25 oder 26, dadurch gekennzeichnet, daß die Zusammensetzung in Form eines Produkts zum Schminken, wie beispielsweise als Make-up, Wangenrouge, Lidschatten, Eyeliner, Mascara oder Lippenstift, oder in Form einer Creme, einer Milch oder eines Serums vorliegt, die als Produkt zur Pflege oder als Sonnenschutzprodukt verwendet werden können.

**28.** Öl-in-Wasser-Emulsion, die enthält:

- (a) eine wäßrige Phase,
- (b) eine Fettphase, die mindestens ein erstes Öl, das unter den flüchtigen Ölen, den $C_{1-20}$-Polyalkylsiloxanen und deren Gemischen ausgewählt ist, in einem Mengenanteil von mindestens 65 Gew.-%, bezogen auf das Gesamtgewicht der Fettphase, und
- (c) ein grenzflächenaktives Silicon, das mindestens eine anionische Gruppe aufweist und ausgewählt ist unter:
  - (i) den grenzflächenaktiven Siliconen mit Phosphatgruppe der Formel I, II, III oder IV:

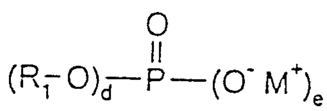

(I)

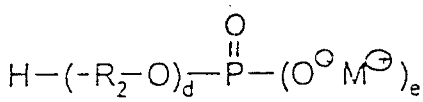 (II)

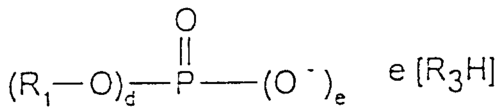 (III)

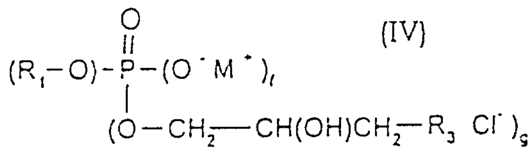 (IV)

worin bedeuten:

$R_1$ die Gruppe der folgenden Formel V:

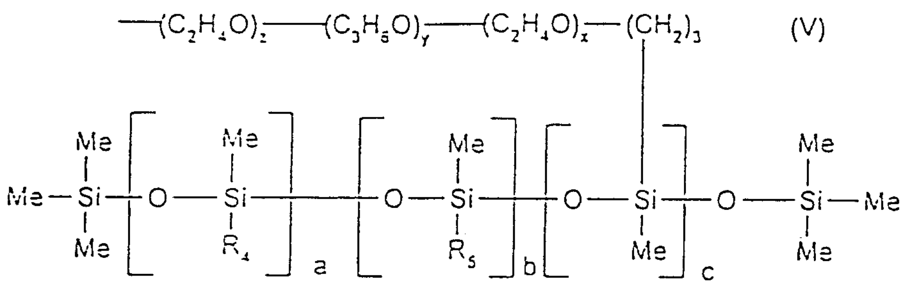 (V)

$R_2$ die Gruppe der folgenden Formel VI

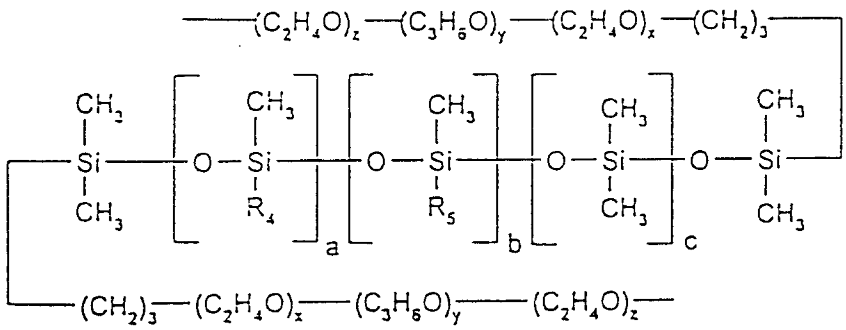 (VI),

wobei in den Formeln bedeuten:

- Me die Methylgruppe,
- $C_2H_4O$ die Gruppe $-CH_2-CH_2-O-$,
- $C_3H_6O$ die Gruppe $-CH_2-CH(CH_3)-O-$,
- a 0 oder eine ganze Zahl im Bereich von 1 bis 200,
- b 0 oder eine ganze Zahl im Bereich von 1 bis 200,
- c eine ganze Zahl im Bereich von 1 bis 200,
- $R_4$ eine Gruppe $-(CH_2)_nCH_3$ oder Phenyl,
  wobei n 0 oder eine ganze Zahl im Bereich von 1 bis 10 bedeutet,

- $R_5$ die Gruppe $-(CH_2)_3-(OCH_2CH_2)_x-(OCH_2CH(CH_3))_y-(OCH_2CH_2)_z-OH$, wobei x, y und z unabhängig voneinander 0 oder ganze Zahlen im Bereich von 1 bis 20 bedeuten,

- $R_3$:

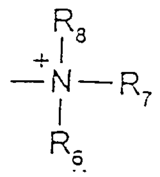

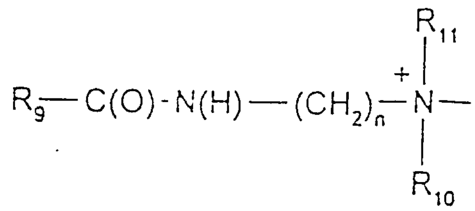

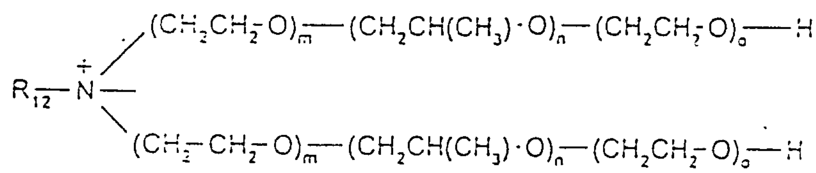

und

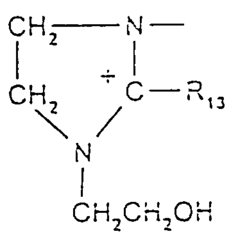

,

worin bedeuten:

- $R_6$ bis $R_9$ unabhängig voneinander eine Alkylgruppe mit 1 bis 20 Kohlenstoffatomen,
- $R_{10}$ und $R_{11}$ unabhängig voneinander eine Alkylgruppe mit 1 bis 3 Kohlenstoffatomen,
- $R_{12}$ und $R_{13}$ unabhängig voneinander eine Alkylgruppe mit 6 bis 20 Kohlenstoffatomen,
- m, n und o unabhängig voneinander 0 oder eine ganze Zahl von 1 bis 20,

- d und e 1 oder 2, mit $d+e=3$,
- f 0 oder 1, g 1 oder 2 und $f+g=2$,
- M H, Na, K, Li, $NH_4$ oder $N(CH_2CH_2OH)_3$,

(ii) den grenzflächenaktiven Siliconen mit Sulfatgruppe der Formel VII:

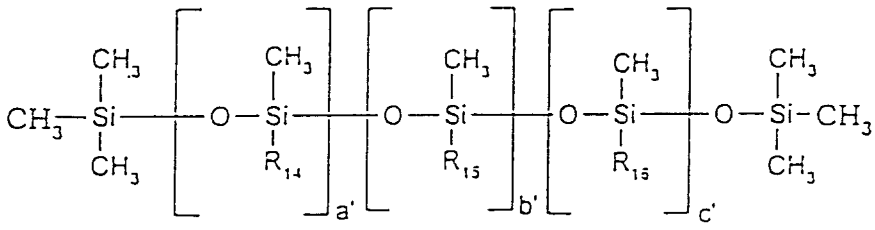 (VII) ,

worin bedeuten:

- $R_{14}$ eine Alkylgruppe mit 1 bis 8 Kohlenstoffatomen oder eine Phenylgruppe,
- $R_{15}$ eine Gruppe $-(CH_2)_3-O-(CH_2CH_2O)_u-(CH_2CH(CH_3)-O)_v-(CH_2CH_2O)_w-SO_3^-M'$, wobei M' unter Na, K, Li oder $NH_4$ ausgewählt ist,
- $R_{16}$ eine Gruppe $-(CH_2)_3-O-(CH_2CH_2O)_u-(CH_2CH(CH_3)-O)_v-(CH_2CH_2O)_w-H$, wobei u, v und w unabhängig voneinander 0 oder eine ganze Zahl im Bereich von 1 bis 100 bedeuten,
- a' und c' unabhängig voneinander 0 oder eine ganze Zahl im Bereich von 1 bis 50,
- b' eine ganze Zahl im Bereich von 1 bis 50,

(iii) den grenzflächenaktiven Siliconen mit Sulfonatgruppe, die durch Umsetzung eines Silicons der Formel VIII:

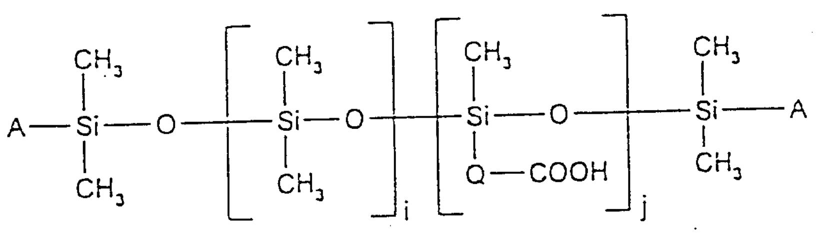 (VIII)

worin bedeuten:
Q $(CH_2)_r$, wobei r eine ganze Zahl im Bereich von 3 bis 17 ist,
j entweder eine ganze Zahl von 1 bis 10 und A eine Methylgruppe oder j = O und A eine Gruppe -Q-COOH,
i eine ganze Zahl im Bereich von 1 bis 200,
mit einem Taurinderivat der Formel
$R_{17}-NH-(CH_2)_2-SO_3M''$ hergestellt werden, worin $R_{17}$ eine Alkylgruppe mit 1 bis 40 Kohlenstoffatomen und M''
Na, K, Li oder $NH_4$ bedeutet,

- (iv) den grenzflächenaktiven Siliconen mit Sulfonatgruppe der Formel IX:

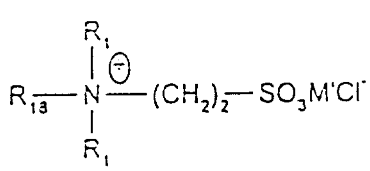 (IX)

worin bedeuten:

$R_{18}$ eine Alkylgruppe mit 1 bis 40 Kohlenstoffatomen,

$R_1$ die oben für die Verbindung der Formel I definierte Gruppe der Formel V,

M' Na, K, Li oder $NH_4$,

mit der Maßgabe, daß die Fettphase der Emulsion eine weitere Fettsubstanz, die von den flüchtigen Ölen und $C_{1-20}$-Polyalkylsiloxanen verschieden ist, in einem Mengenanteil von höchstens 7 Gew.-%, bezogen auf das Gesamtgewicht der Emulsion, enthält, wenn das grenzflächenaktive Silicon die Formel I aufweist.

**29.** Emulsion nach Anspruch 28, dadurch gekennzeichnet, daß das flüchtige Öl unter den flüchtigen Kohlenwasserstoffölen, den flüchtigen Siliconölen und deren Gemischen ausgewählt ist.

**30.** Emulsion nach einem der Ansprüche 28 und 29, dadurch gekennzeichnet, daß das flüchtige Öl unter den Isoparaffinen, den cyclischen flüchtigen Siliconen mit 3 bis 8 Siliciumatomen und vorzugsweise 4 bis 6 Siliciumatomen, den Cyclocopolymeren vom Dimethylsiloxan/Methylalkylsiloxan-Typ, den geradkettigen flüchtigen Siliconen mit 2 bis 9 Siliciumatomen und deren Gemischen ausgewählt ist.

**31.** Emulsion nach einem der Ansprüche 28 bis 30, dadurch gekennzeichnet, daß das flüchtige Öl unter Isododecan, Cyclotetradimethylsiloxan, Cyclopentadimethylsiloxan, Cyclohexadimethylsiloxan, Dimethylsiloxan/Methyloctylsiloxan-Cyclocopolymer, Hexamethyldisiloxan, Hexylheptamethyltrisiloxan, Octylheptamethyltrisiloxan und deren Gemischen ausgewählt ist.

**32.** Emulsion nach einem der Ansprüche 28 bis 31, dadurch gekennzeichnet, daß die $C_{1-20}$-Polyalkylsiloxane eine Viskosität bei 25 °C von höchstens 0,06 $m^2/s$ aufweisen.

**33.** Emulsion nach einem der Ansprüche 28 bis 32, dadurch gekennzeichnet, daß die $C_{1-20}$-Polyalkylsiloxane unter den geradkettigen Polydimethylsiloxanen, den Alkylmethylpolysiloxanen und deren Gemischen ausgewählt sind.

**34.** Emulsion nach einem der Ansprüche 28 bis 33, dadurch gekennzeichnet, daß die Fettphase mindestens 75 Gew.-% Öl, das unter den flüchtigen Ölen, den $C_{1-20}$-Polyalkylsiloxanen und deren Gemischen ausgewählt ist, bezogen auf das Gesamtgewicht der Fettphase, enthält.

**35.** Emulsion nach einem der Ansprüche 28 bis 34, dadurch gekennzeichnet, daß die Fettphase 100 Gew.-% erstes Öl, bezogen auf das Gesamtgewicht der Fettphase, enthält, wenn das grenzflächenaktive silicon von den grenzflächenaktiven Siliconen der Formel I verschieden ist.

**36.** Emulsion nach einem der Ansprüche 28 bis 35, dadurch gekennzeichnet, daß die Fettphase ferner mindestens eine weitere Fettsubstanz, die von dem ersten Öl verschieden ist, in einem Mengenanteil von höchstens 7 Gew.-%, bezogen auf das Gesamtgewicht der Emulsion, enthält.

**37.** Emulsion nach einem der Ansprüche 28 bis 36, dadurch gekennzeichnet, daß die andere Fettsubstanz unter den pflanzlichen, mineralischen, tierischen und/oder synthetischen Ölen, pastösen Fettsubstanzen, Gummen und/oder Wachsen ausgewählt ist.

**38.** Emulsion nach einem der Ansprüche 28 bis 37, dadurch gekennzeichnet, daß sie 5 bis 40 Gew.-% Fettphase, 0,5 bis 15 Gew.-% grenzflächenaktives Silicon mit anionischer Gruppe und 15 bis 94,5 Gew.-% wäßrige Phase, bezogen auf das Gesamtgewicht der Emulsion, enthält.

**39.** Emulsion nach einem der Ansprüche 28 bis 37, dadurch gekennzeichnet, daß sie 12 bis 30 Gew.-% Fettphase, 3 bis 6 Gew.-% grenzflächenaktives Silicon mit anionischer Gruppe und 40 bis 70 Gew.-% wäßrige Phase, bezogen auf das Gesamtgewicht der Emulsion, enthält.

**40.** Kosmetische, pharmazeutische oder hygienische Zusammensetzung, dadurch gekennzeichnet, daß sie eine Emulsion nach einem der Ansprüche 28 bis 39 enthält.

**41.** Zusammensetzung nach Anspruch 40, dadurch gekennzeichnet, daß sie in Form eines Produkts zum Schminken, wie als Make-up, Wangenrouge, Lidschatten, Eyeliner, Mascara oder Lippenstift oder in Form einer Creme, einer Milch oder eines Serums vorliegt, die als Produkt zur Pflege oder als Sonnenschutznittel verwendet werden können.

**42.** Verfahren zur nicht therapeutischen Behandlung der Haut oder der Kopfhaut, dadurch gekennzeichnet, daß auf die

Haut und/oder die Kopfhaut eine kosmetische Emulsion nach einem der Ansprüche 28 bis 39 und/oder eine kosmetische Zusammensetzung nach einem der Ansprüche 40 und 41 aufgetragen wird.

**43.** Verfahren zum Schminken der Haut und/oder der Kopfhaut, dadurch gekennzeichnet, daß auf die Haut und/oder die Kopfhaut eine kosmetische Emulsion nach einem der Ansprüche 28 bis 37 und/oder eine kosmetische Zusammensetzung nach einem der Ansprüche 40 bis 41 aufgetragen wird.